# EUROPEAN PATENT SPECIFICATION

(11) **EP 4 044 916 B1**
(45) Date of publication and mention of the grant of the patent: **31.12.2025**
(21) Application number: 20781245.4
(22) Date of filing: 16.09.2020
(51) Int. Cl.: A61B 5/08, A61B 5/0205, A61B 5/00

(54) **DETECTING ONE OR MORE PATIENT COUGHS BASED ON AN ELECTROGRAM SIGNAL AND AN ACCELEROMETER SIGNAL**
ERKENNUNG EINES ODER MEHRERER HUSTENANFÄLLE VON PATIENTEN BASIEREND AUF EINEM ELEKTROGRAMMSIGNAL UND EINEM BESCHLEUNIGUNGSMESSERSIGNAL
DÉTECTION DE LA TOUX D'UN PATIENT SUR LA BASE D'UN SIGNAL D'ÉLECTROGRAMME ET D'UN SIGNAL D'ACCÉLÉROMÈTRE

(30) Priority: 14.10.2019 US 201916601056
(43) Date of publication of application: 24.08.2022
(73) Proprietor: Medtronic, Inc., Minneapolis, Minnesota 55432 (US)
(72) Inventor: GUNDERSON, Bruce D., Minneapolis, MN 55432 (US)
(74) Representative: Zimmermann & Partner Patentanwälte mbB
(86) International application number: PCT/US2020/050980
(87) International publication number: WO 2021/076257

(56) References cited:
- WO-A1-2016/014135
- JP-A- 2008 173 280
- JP-A- H0 998 964
- US-A1- 2007 276 278
- US-A1- 2011 245 688
- US-A1- 2012 302 898
- US-B2- 7 727 161
- THOMAS DRUGMAN ET AL: "Objective Study of Sensor Relevance for Automatic Cough Detection", IEEE JOURNAL OF BIOMEDICAL AND HEALTH INFORMATICS, IEEE, PISCATAWAY, NJ, USA, vol. 17, no. 3, 1 May 2013 (2013-05-01), pages 699 - 707, XP011506359, ISSN: 2168-2194, DOI: 10.1109/JBHI.2013.2239303

## Description

### TECHNICAL FIELD

The disclosure relates generally to medical device systems and, more particularly, medical device systems configured to monitor patient parameters.

### BACKGROUND

Some types of medical devices may be used to monitor one or more physiological parameters of a patient. Such medical devices may include, or may be part of a system that includes, sensors that detect signals associated with such physiological parameters. Values determined based on such signals may be used to assist in detecting changes in patient conditions, in evaluating the efficacy of a therapy, or in generally evaluating patient health. Document JP 2008 173280 A describes a cough detecting system using an electrocardiograph and a body motion detection unit such that cough can be detected based on at least one of the body movement signals, or cough can be detected based on both signals.

### SUMMARY

In general, the disclosure is directed to devices, systems, and techniques for using a medical device to collect an electrogram (EGM) signal indicative of one or more physiological signals of a patient and an accelerometer signal indicative of one or more movements of the patient. The EGM signal and the accelerometer signal may allow processing circuitry to determine when the patient coughs. In this way, the processing circuitry may track a rate in which the patient coughs over a period of time, If the rate in which the patient coughs changes over the period of time, the processing circuitry may determine that the patient is experiencing one or more patient conditions such as chronic obstructive pulmonary disease (COPD) or experiencing an exacerbation of one or more patient conditions such as COPD.

EGM signals, in some cases, may indicate one or more events of a heart cycle such as ventricular depolarizations and/or repolarizations, atrial depolarizations and/or repolarizations, or any combination thereof. Such EGMs may be referred to as cardiac EGMs. Additionally, EGM signals may include noise from various sources including noise relating to a cough by the patient. In other words, if a patient coughs, the cough may be reflected in the EGM signal collected by the medical device. However, it may be the case that not all noise in the EGM signal relates to cough(s) by the patient. As such, it may be beneficial to analyze the accelerometer signal to determine whether the accelerometer signal, in addition to the EGM signal, indicates a cough. For example, processing circuitry may analyze a section of accelerometer signal which corresponds to a section of EGM signal which indicates a cough. If the accelerometer signal also indicates a cough, the processing circuitry may determine that a cough occurred at a time in which the section of the EGM signal and the section of the accelerometer signal are recorded by the medical device.

In some examples, the accelerometer signal includes a vertical component, a lateral component, and a frontal component corresponding to a vertical axis, a lateral axis, and a frontal axis, respectively. In this way, the accelerometer signal represents a three-dimensional measurement of acceleration. It may be beneficial to analyze the frontal component of the accelerometer signal to determine whether the accelerometer signal indicates a cough. For example, when coughing, a patient may lean slightly forward sharply, causing the medical device to move along the frontal axis, the forward movement being reflected in the frontal component of the accelerometer signal. In response to the processing circuitry detecting noise in the EGM signal indicative of a cough and detecting a forward movement in the frontal component of the accelerometer signal, the processing circuitry may determine that a cough occurred.

The techniques of this disclosure may provide one or more advantages. For example, detecting one or more patient coughs based on both of the EGM signal and the accelerometer signal collected by a medical device may be more accurate than detecting coughs without using one or both of the EGM signal and the accelerometer signal. More specifically, it may be beneficial to detect portions of the EGM signal which possibly indicate a cough, and subsequently analyze corresponding portions of the accelerometer signal in order to confirm that the portions of the EGM signal indeed indicate a cough. It may be unlikely that both of a portion of the EGM signal and a corresponding portion of the accelerometer signal indicate a cough when a cough did not occur during a period of time in which the portion of the EGM signal and the corresponding portion of the accelerometer signal are collected by the medical device. Additionally, it may be beneficial to analyze the frontal component of the accelerometer system when detecting coughs using the accelerometer signal since the patient moves their chest forward along the frontal axis during a cough.

The claimed subject-matter is directed to a medical device system according to claim 1 and a non-transitory computer-readable medium according to claim 11.

In some examples, a method includes collecting, using a plurality of electrodes of a medical device, an EGM signal, where the EGM signal is indicative of one or more muscle movements that occur during a cough, collecting, using an accelerometer of the medical device, an accelerometer signal, where the accelerometer signal is indicative of one or more patient movements that occur during a cough, identifying, in the EGM signal, a segment of the EGM signal including noise indicative of a muscle movement occurring during a cough, identifying, in response to identifying the segment of the EGM signal, a segment of the accelerometer signal which is collected over a period of time, where the period of time in which the accelerometer signal is collected corresponds to a period of time in which the segment of the EGM signal is collected, determining whether a parameter value associated with the segment of the accelerometer signal is greater than a threshold parameter value, and incrementing, in response to the parameter value associated with the segment of the accelerometer signal being greater than the threshold parameter value, a cough count value.

The summary is intended to provide an overview of the subject matter described in this disclosure. It is not intended to provide an exclusive or exhaustive explanation of the systems, device, and methods described in detail within the accompanying drawings and description below. Further details of one or more examples of this disclosure are set forth in the accompanying drawings and in the description below. Other features, objects, and advantages will be apparent from the description and drawings, and from the claims.

### BRIEF DESCRIPTION OF DRAWINGS

FIG. 1 illustrates the environment of an example medical device system in conjunction with a patient, in accordance with one or more techniques of this disclosure.
FIG. 2 is a conceptual drawing illustrating an example configuration of the implantable medical device (IMD) of the medical device system of FIG. 1, in accordance with one or more techniques described herein.
FIG. 3 is a functional block diagram illustrating an example configuration of the IMD of FIGS. 1 and 2, in accordance with one or more techniques described herein.
FIGS. 4A and 4B are block diagrams illustrating two additional example IMDs that may be substantially similar to the IMD of FIGS. 1-3, but which may include one or more additional features, in accordance with one or more techniques described herein.
FIG. 5 is a block diagram illustrating an example configuration of components of the external device of FIG. 1, in accordance with one or more techniques of this disclosure.
FIG. 6 is a block diagram illustrating an example system that includes an access point, a network, external computing devices, such as a server, and one or more other computing devices, which may be coupled to the IMD of FIGS. 1-4, an external device, and processing circuitry via a network, in accordance with one or more techniques described herein.
FIG. 7 is a graph illustrating a first electrogram (EGM) plot, a second EGM plot, and a third EGM plot, in accordance with one or more techniques of this disclosure.
FIG. 8 is a graph illustrating a set of physiological signal plots recorded concurrently, in accordance with one or more techniques of this disclosure.
FIG. 9 is a graph illustrating a far-field EGM plot and a near-field EGM plot, in accordance with one or more techniques of this disclosure.
FIG. 10 is a graph illustrating a sequence of EGM samples representing an EGM signal, in accordance with one or more techniques of this disclosure.
FIG. 11 is a graph illustrating a group of EGM samples, in accordance with one or more techniques of this disclosure.
FIG. 12 is a graph illustrating a first accelerometer signal component plot, a second accelerometer signal component plot, and a third accelerometer signal component plot, in accordance with one or more techniques of this disclosure.
FIG. 13 is a flow diagram illustrating an example operation for detecting one or more coughs of a patient, in accordance with one or more techniques of this disclosure.
FIG. 14 is a flow diagram illustrating an example operation for identifying a segment of an EGM signal which includes noise that may be indicative of one or more coughs, in accordance with one or more techniques of this disclosure.

Like reference characters denote like elements throughout the description and figures.

### DETAILED DESCRIPTION

This disclosure describes techniques for detecting coughs of a patient in order to track one or more patient conditions. Changes in cough frequency may be a sign of a change in a patient condition. An increased coughing frequency detected in a patient may indicate an exasperation in need of one or more medical treatments. For example, coughing frequency may provide an important metric for monitoring one or more patient conditions such as Chronic Obstructive Pulmonary Disease (COPD). Data collected by an implantable medical device (IMD), or one or more implantable or external devices, may be used detect coughs and determine coughing frequency. For example, an IMD, or one or more other devices, may be configured to record an electrogram (EGM) signal and an accelerometer signal, both of which may include information that may be analyzed to detect coughs.

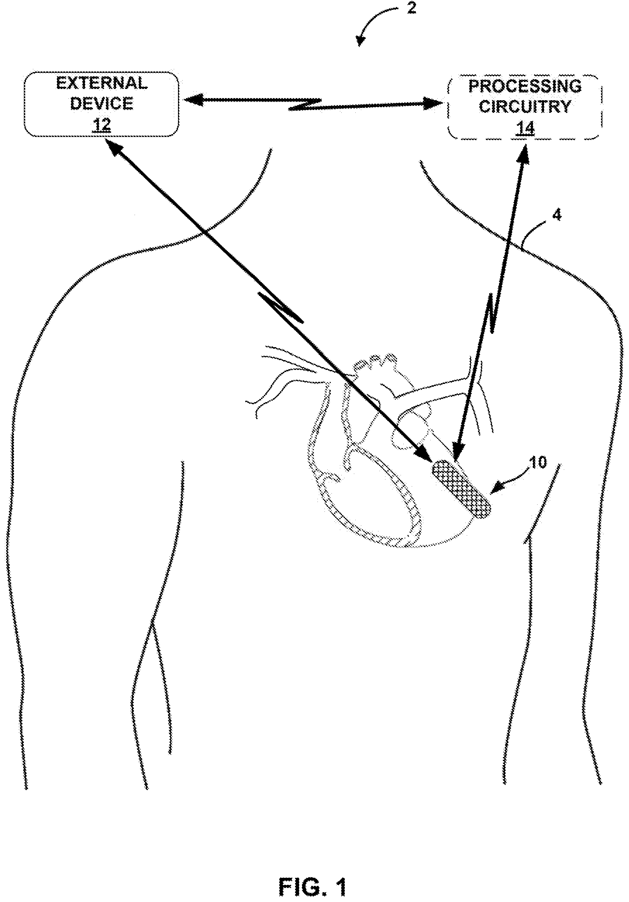

FIG. 1 illustrates the environment of an example medical device system 2 in conjunction with a patient 4, in accordance with one or more techniques of this disclosure. The example techniques may be used with an IMD 10, which may be in wireless communication with at least one of external device 12 and other devices not pictured in FIG. 1. Processing circuitry 14 is conceptually illustrated in FIG. 1 as separate from IMD 10 and external device 12, but may be processing circuitry of IMD 10 and/or processing circuitry of external device 12. In general, the techniques of this disclosure may be performed by processing circuitry 14 of one or more devices of a system, such as one or more devices that include sensors that provide signals, or processing circuitry of one or more devices that do not include sensors, but nevertheless analyze signals using the techniques described herein. For example, another external device (not pictured in FIG. 1) may include at least a portion of processing circuitry 14, the other external device configured for remote communication with IMD 10 and/or external device 12 via a network.

In some examples, IMD 10 is implanted outside of a thoracic cavity of patient 4 (e.g., subcutaneously in the pectoral location illustrated in FIG. 1). IMD 10 may be positioned near the sternum near or just below the level of patient 4's heart, e.g., at least partially within the cardiac silhouette. In some examples, IMD 10 takes the form of a LINQ^{™} Insertable Cardiac Monitor (ICM), available from Medtronic plc, of Dublin, Ireland.

Clinicians sometimes diagnose patients with medical conditions based on one or more observed physiological signals collected by physiological sensors, such as electrodes, optical sensors, chemical sensors, temperature sensors, acoustic sensors, and motion sensors. In some cases, clinicians apply non-invasive sensors to patients in order to sense one or more physiological signals while a patient is in a clinic for a medical appointment. However, in some examples, physiological markers (e.g., irregular heartbeats and long-term respiration trends) of a patient condition are rare or are difficult to observe over a relatively short period of time. As such, in these examples, a clinician may be unable to observe the physiological markers needed to diagnose a patient with a medical condition while monitoring one or more physiological signals of the patient during a medical appointment. In the example illustrated in FIG. 1, IMD 10 is implanted within patient 4 to continuously record one or more physiological signals of patient 4 over an extended period of time.

In some examples, IMD 10 includes a plurality of electrodes. The plurality of electrodes is configured to detect signals that enable processing circuitry 14, e.g., of IMD 10, to determine current values of additional parameters associated with the cardiac and/or lung functions of patient 4. In some examples, the plurality of electrodes of IMD 10 are configured to detect a signal indicative of an electric potential of the tissue surrounding the IMD 10. Moreover, IMD 10 may additionally or alternatively include one or more optical sensors, accelerometers, temperature sensors, chemical sensors, light sensors, pressure sensors, in some examples. Such sensors may detect one or more physiological parameters indicative of a patient condition.

External device 12 may be a hand-held computing device with a display viewable by the user and an interface for providing input to external device 12 (i.e., a user input mechanism). For example, external device 12 may include a small display screen (e.g., a liquid crystal display (LCD) or a light emitting diode (LED) display) that presents information to the user. In addition, external device 12 may include a touch screen display, keypad, buttons, a peripheral pointing device, voice activation, or another input mechanism that allows the user to navigate through the user interface of external device 12 and provide input. If external device 12 includes buttons and a keypad, the buttons may be dedicated to performing a certain function, e.g., a power button, the buttons and the keypad may be soft keys that change in function depending upon the section of the user interface currently viewed by the user, or any combination thereof.

In other examples, external device 12 may be a larger workstation or a separate application within another multi-function device, rather than a dedicated computing device. For example, the multi-function device may be a notebook computer, tablet computer, workstation, one or more servers, cellular phone, personal digital assistant, or another computing device that may run an application that enables the computing device to operate as a secure device.

When external device 12 is configured for use by the clinician, external device 12 may be used to transmit instructions to IMD 10. Example instructions may include requests to set electrode combinations for sensing and any other information that may be useful for programming into IMD 10. The clinician may also configure and store operational parameters for IMD 10 within IMD 10 with the aid of external device 12. In some examples, external device 12 assists the clinician in the configuration of IMD 10 by providing a system for identifying potentially beneficial operational parameter values.

Whether external device 12 is configured for clinician or patient use, external device 12 is configured to communicate with IMD 10 and, optionally, another computing device (not illustrated in FIG. 1), via wireless communication. External device 12, for example, may communicate via near-field communication technologies (e.g., inductive coupling, NFC or other communication technologies operable at ranges less than 10-20 cm) and far-field communication technologies (e.g., RF telemetry according to the 802.11 or Bluetooth^{®} specification sets, or other communication technologies operable at ranges greater than near-field communication technologies).

Processing circuitry 14, in some examples, may include one or more processors that are configured to implement functionality and/or process instructions for execution within IMD 10. For example, processing circuitry 14 may be capable of processing instructions stored in a storage device. Processing circuitry 14 may include, for example, microprocessors, digital signal processors (DSPs), application specific integrated circuits (ASICs), field-programmable gate arrays (FPGAs), or equivalent discrete or integrated logic circuitry, or a combination of any of the foregoing devices or circuitry. Accordingly, processing circuitry 14 may include any suitable structure, whether in hardware, software, firmware, or any combination thereof, to perform the functions ascribed herein to processing circuitry 14.

Processing circuitry 14 may represent processing circuitry located within any combination of IMD 10 and external device 12. In some examples, processing circuitry 14 may be entirely located within a housing of IMD 10. In other examples, processing circuitry 14 may be entirely located within a housing of external device 12. In other examples, processing circuitry 14 may be located within any combination of IMD 10, external device 12, and another device or group of devices that are not illustrated in FIG. 1. As such, techniques and capabilities attributed herein to processing circuitry 14 may be attributed to any combination of IMD 10, external device 12, and other devices that are not illustrated in FIG. 1.

Medical device system 2 of FIG. 1 is an example of a system for collecting an EGM signal according to one or more techniques of this disclosure. In some examples, processing circuitry 14 includes EGM analysis circuitry configured to determine one or more parameters of an EGM signal of patient 4. In one example, an EGM signal is sensed via one or more electrodes of IMD 10. An EGM is a signal representative of electrical activity of the heart, measured by electrodes implanted within the body, and often within the heart itself. For example, a cardiac EGM may include P-waves (depolarization of the atria), R-waves (depolarization of the ventricles), and T-waves (repolarization of the ventricles), among other events. Information relating to the aforementioned events, such as time separating one or more of the events, may be applied for a number of purposes, such as to determine whether an arrhythmia is occurring and/or predict whether an arrhythmia is likely to occur. Cardiac signal analysis circuitry, which may be implemented as part of processing circuitry 14, may perform signal processing techniques to extract information indicating the one or more parameters of the cardiac signal.

Additionally, the EGM signal may include noise that is introduced into the EGM signal by a variety of cardiac and non/cardiac sources. In some examples, muscle noise may be recorded on one or more leads of a medical device during outer insulation breaches near an implant sight. Additionally, muscle noise may be recorded using far-field electrode configurations (e.g., a configuration using a right ventricle (RV) coil and a housing of the medical device). In some examples, IMD 10 is implanted near one or more muscles contributing to a cough. Respiratory muscles used during a cough may include abdominal muscles, intercostal muscles, and the diaphragm. For example, the abdominal muscles and the intercostal muscles may contract during a cough and the diaphragm may relax during a cough. The EGM signal recorded by IMD 10 may include muscle signals corresponding to a cough by patient 4. In some cases, the muscle signals reflected in the EGM signal recorded by IMD 10 may be referred to herein as "muscle noise." For example, IMD 10 may detect the muscle noise in the EGM signal due to contractions in the muscles contributing to the cough, where the muscle noise does not originate from the heart of patient 4. In this way, processing circuitry 14 may analyze the EGM signal recorded by IMD 10 in order to identify sections of the EGM signal which include the muscle noise that indicates a contraction of muscles due to a cough. In this way, the EGM signal may indicate one or more occurrences of a cough performed by patient 4. One or more algorithms executed by processing circuitry 14 may detect myopotential and electromagnetic interference (EMI) noise, which may be types of noise introduced into the EGM signal due to a cough. In some cases, muscle noise from a cough may be differentiated from other sources of noise. For example, upper body movements, which may be recorded using an accelerometer of IMD 10 during a cough, may be used by processing circuitry 14 to differentiate muscle noise from other sources of noise.

In some examples, IMD 10 includes one or more accelerometers. An accelerometer of IMD 10 may collect an accelerometer signal which reflects a measurement of a motion of patient 4. In some cases, the accelerometer may collect a three-axis accelerometer signal indicative of patient 4's movements within a three-dimensional Cartesian space. For example, the accelerometer signal may include a vertical axis accelerometer signal vector, a lateral axis accelerometer signal vector, and a frontal axis accelerometer signal vector. The vertical axis accelerometer signal vector may represent an acceleration of patient 4 along a vertical axis, the lateral axis accelerometer signal vector may represent an acceleration of patient 4 along a lateral axis, and the frontal axis accelerometer signal vector may represent an acceleration of patient 4 along a frontal axis. In some cases, the vertical axis substantially extends along a torso of patient 4 when patient 4 from a neck of patient 4 to a waist of patient 4, the lateral axis extends across a chest of patient 4 perpendicular to the vertical axis, and the frontal axis extends outward from and through the chest of patient 4, the frontal axis being perpendicular to the vertical axis and the lateral axis.

In some examples, processing circuitry 14 may be configured to identify, in an EGM signal collected by IMD 10, a segment of the EGM signal that includes noise indicative of a muscle movement occurring during a cough. For example, the muscle noise identified in the EGM signal may be introduced into the EGM signal be contractions in one or both of the diaphragm and the intercostal muscles of patient 4 caused by a cough. During a cough, one or both of the diaphragm and the intercostal muscles contractions may sharply contract. Since IMD 10 may be implanted close to a chest cavity of patient 4, the EGM recorded by IMD 10 may include muscle noise indicative of coughs.

The EGM signal recorded by IMD 10 may include a first sequence of EGM samples. For example, IMD 10 may collect EGM samples at a predetermined sampling rate in order to collect the first sequence of EGM samples. Additionally, processing circuitry 14 may be configured to generate a second sequence of EGM samples, where the second sequence of EGM samples represents a derivative of the first sequence of EGM samples. In this way, processing circuitry 14 may be configured to calculate the derivative of the EGM signal. In some examples, it may be beneficial for processing circuitry 14 to calculate the derivative of the EGM signal in order to set the EGM signal around a baseline axis (e.g., remove baseline noise from the EGM signal). Additionally, it may be easier for processing circuitry 14 to detect cardiac depolarization events in the derivative of EGM signal to detect cardiac depolarization events in the EGM signal before the derivative is calculated. Processing circuitry 14 may be configured to identify portions of the EGM signal which indicate coughs of patient 4.

One way in which processing circuitry 14 may identify coughs is to monitor the EGM signal for "slope reversals," events in which a slope of the EGM signal switches signs from a first pair of consecutive EGM samples to a second pair of consecutive EGM samples immediately following the first pair of consecutive EGM samples. Additionally, a "slope change event" may be referred to herein as any event in which a slope of the EGM signal changes sign (e.g., changes from positive to negative or changes from negative to positive) from a first pair of consecutive EGM samples to a second pair of consecutive EGM samples immediately following the first pair of consecutive EGM samples. In this way, a slope reversal may be a different classification than a slope change event. For example, a slope change event may include an R-wave (e.g., a ventricular depolarization). A ventricular depolarization may cause a significant change in the EGM signal and trigger a slope change event. However, a magnitude associated with the slope change event associated with the R-wave may be relatively large compared with other slope change events. As such, processing circuitry 14 may determine that the R-wave is not a slope reversal, and therefore not related to muscle contractions relating to a cough.

Processing circuitry 14 may determine a set of slope reversals in a portion of the second sequence of EGM samples. In some examples, the portion of the second sequence of EGM samples may represent a portion of the second sequence of EGM samples occurring after an R-wave. For example, processing circuitry 14 may detect an R-wave in the EGM signal and analyze a portion of the second sequence of EGM samples for slope reversals following the R-wave. Additionally, or alternatively, in some examples, processing circuitry 14 may analyze the second sequence of EGM samples according to a sliding one-second window where each second of data is analyzed to detect noise that may be related to one or more coughs by patient 4 in the respective one-second segment of EGM samples. Processing circuitry 14 may identify slope reversals as being events that are potentially related to contractions of one or more muscles due to a cough and not related to cardiac events or other types of noise.

The second sequence of EGM samples may represent a derivative, or a "difference" of the first sequence of EGM samples (e.g., the EGM signal collected by IMD 10). As such, a magnitude of an EGM sample of the second sequence of EGM samples which occurs at a point in time may represent a slope of the EGM signal collected by IMD 10 at the point in time. Moreover, a "zero crossing" in the second sequence of EGM samples may represent a point of zero slope (e.g., a peak or a valley) in the EGM signal collected by IMD 10. In this way, a zero crossing in the second sequence of EGM samples may represent a slope change event in the first sequence of EGM samples, a slope change event being a point at which a slope of the first sequence of EGM samples changes from positive to negative or negative to positive. Processing circuitry 14, in some cases, may determine a slope change event in the first sequence of EGM samples to be an event in which a pair of consecutive EGM samples of the second sequence of EGM samples changes sign. For example, if an amplitude of a first EGM sample of the pair of consecutive EGM samples is positive and a second EGM sample of the pair of consecutive EGM samples is negative, the pair of consecutive EGM samples may represent a slope change event. Additionally, or alternatively, if the first EGM sample is positive and the second EGM sample is negative, the pair of consecutive EGM samples may represent a slope change event.

In some cases, processing circuitry 14 might not classify at least some slope change events as slope reversals. For example, slope change events that are outside of a range of intensity values might not be classified as slope reversals. In order to determine whether a slope change event is a slope reversal, processing circuitry 14 may calculate an intensity value corresponding to each slope change event detected by processing circuitry 14. The intensity value, in some cases, may be a difference between an amplitude of a first EGM sample and an amplitude of a second EGM sample, where the first EGM sample and the second EGM sample represent a pair of consecutive EGM samples of the second sequence of EGM samples that processing circuitry 14 identifies as a slope change event in the EGM signal collected by IMD 10. If the intensity value of the slope change event is within a range from a first threshold intensity value to a second threshold intensity value, processing circuitry 14 may determine that the slope change event is a slope reversal that is potentially related to a cough of patient 4. On the other hand, if the intensity value of the slope change event is outside of the range from the first threshold intensity value to the second threshold intensity value, processing circuitry 14 may determine that the slope change event is not a slope reversal, and thus not related to a cough of patient 4. For example, processing circuitry 14 may identify an R-wave as a slope change event. However, an intensity value associated with the R-wave may be greater than the second threshold intensity value and processing circuitry 14 may determine that the R-wave does not represent a slope reversal and thus is not related to noise arising from a cough by patient 4.

Processing circuitry 14 may determine if a number of slope reversals detected in a segment of the second sequence of EGM samples is greater than a threshold number of slope reversals. In some examples, the threshold number of slope reversals is within a range from 3 slope reversals to 20 slope reversals. If the number of slope reversals is not greater than the threshold number of slope reversals, processing circuitry 14 may select another segment of the second sequence of EGM samples for analysis to detect a presence of noise relating to a cough of patient 4. On the other hand, if the number of slope reversals is greater than the threshold number of slope reversals, processing circuitry 14 may determine the intensity value corresponding to each slope reversal of the set of slope reversals identified in the segment of the second sequence of EGM samples. After determining the intensity value corresponding to each slope reversal of the set of slope reversals, processing circuitry 14 may calculate a slope reversal intensity parameter value corresponding to the set of slope reversals.

In some examples, the slope reversal intensity parameter value represents a median intensity value of the set of slope reversals. In some examples, the slope reversal intensity parameter value represents a sum of the respective intensity values corresponding to each slope reversal of the set of slope reversals. Processing circuitry 14 may determine whether a slope reversal intensity parameter value is greater than a threshold slope reversal intensity parameter value. If processing circuitry 14 determines the slope reversal intensity parameter value is not greater than the threshold slope reversal intensity parameter value, processing circuitry 14 selects another segment of the second sequence of EGM samples. If processing circuitry 14 determines the slope reversal intensity parameter value is greater than the threshold slope reversal intensity parameter value, processing circuitry 14 identifies the segment of the second sequence of EGM samples as a segment including noise indicative of a muscle movement occurring during a cough.

Processing circuitry 14 may be configured to identify, in response to identifying the segment of the second sequence of EGM samples, a segment of the accelerometer signal which is collected over a period of time. The period of time in which the accelerometer signal is collected by IMD 10 may correspond to a period of time in which the segment of the EGM signal is collected by IMD 10. In some examples, the period of time in which the segment of the accelerometer signal is collected is the same as the period of time in which the segment of the EGM signal is collected. In some examples, at least a portion of the period of time in which the segment of the accelerometer signal is collected overlaps with at least a portion of the period of time in which the segment of the EGM signal is collected. As such, processing circuitry 14 may analyze the EGM signal and the accelerometer signal during a window of time that possibly includes noise indicative of one or more coughs. If both of the accelerometer signal and the EGM signal indicate a cough, processing circuitry 14 may determine that one or more coughs occurred during the window of time.

For example, processing circuitry 14 may determine whether a parameter value associated with the segment of the accelerometer signal is greater than a threshold parameter value. In some examples, the accelerometer signal includes a vertical component, a lateral component, and a frontal component corresponding to a vertical axis, a lateral axis, and a frontal axis, respectively. In this way, the accelerometer signal represents a three-dimensional measurement of acceleration. The vertical axis, the lateral axis, and the frontal axis may represent three axes of a Cartesian space. In this way, the accelerometer signal may track movements of patient 4 within a three-dimensional space. It may be beneficial for processing circuitry 14 to analyze the frontal component of the accelerometer signal in order to determine whether the patient coughed during the period of time in which the segment of the accelerometer signal is being collected. For example, the vertical axis of the accelerometer signal may extend along a torso of patient 4 roughly along a spine of patient 4. The lateral axis may extend, perpendicular to the vertical axis, across a chest of patient 4. Additionally, the frontal axis may extend outwards from the chest of patient 4 perpendicular to the vertical axis and perpendicular to the lateral axis. During a cough, the chest of patient 4 may move forwards along the frontal axis. In this way, the chest movement which occurs due to a cough may be recorded in the frontal component of the accelerometer signal. In this way, the parameter value of the segment of the accelerometer signal may correspond to a magnitude value of the frontal component of the accelerometer signal. Additionally, or alternatively, in some examples, the parameter value may represent one or more of a derivative of the frontal component of the segment of the accelerometer signal, an area under the frontal component of the accelerometer signal, or another parameter corresponding to the frontal component, the vertical component, or the lateral component of the accelerometer signal.

Processing circuitry 14 may increment, in response to the parameter value associated with the segment of the accelerometer signal being greater than the threshold parameter value, a cough count value. In some examples, processing circuitry 14 may attach a time stamp to the detected cough so that processing circuitry 14 may determine a time in which each detected cough occurs. In some cases, processing circuitry 14 is configured to determine, based on the cough count value, a cough rate associated with the patient, where the cough rate represents a number of coughs detected per unit time. Processing circuitry 14 may be configured to track the cough rate associated with patient 4 over a period of time. In this way, processing circuitry 14 may identify one or more trends in the cough rate over the period of time. In some cases, if the cough rate increases from a first point in time to a second point in time, processing circuitry 14 may determine, that a patient condition (e.g., COPD) is occurring and/or worsening. In some examples, processing circuitry 14 is further configured to output an alert indicting the occurrence and/or the worsening of the patient condition identified by processing circuitry 14.

In some examples, to identify the one or more trends in the cough rate, processing circuitry 14 may perform a statistical process control (SPC) based on cough rate data over a period of time. For example, cough rate data may include a set of cough rate values that are collected over the period of time. Processing circuitry 14 may determine a baseline cough rate value based on the set of cough rate values. If a cough rate value of the set of cough rate values is greater than the baseline cough rate value by more than a threshold cough rate difference value, processing circuitry 14 may determine that a worsening of one or more patient conditions (e.g., COPD) occurs at a time in which the cough rate value is measured.

In some examples, it may be beneficial to detect coughing exacerbations in order to manage one or more patient conditions, such as COPD. For example, it may be beneficial to track a coughing frequency of patient 4 over a period of time lasting days or weeks. Processing circuitry 14 may detect acute exacerbation in coughing, allowing patient 4 to receive treatment for such a condition. Coughing exacerbations may be caused by a respiratory infection, air pollution or, other triggers of lung inflammation.

Although in one example IMD 10 takes the form of an ICM, in other examples, IMD 10 takes the form of any combination of implantable cardioverter defibrillators (ICDs) with intravascular or extravascular leads, pacemakers, cardiac resynchronization therapy devices (CRT-Ds), neuromodulation devices, left ventricular assist devices (LVADs), implantable sensors, orthopedic devices, or drug pumps, as examples. Moreover, techniques of this disclosure may be used to detect one or more coughs of patient 4 based on an ECG signal and/or an accelerometer signal collected by one or more of the aforementioned devices.

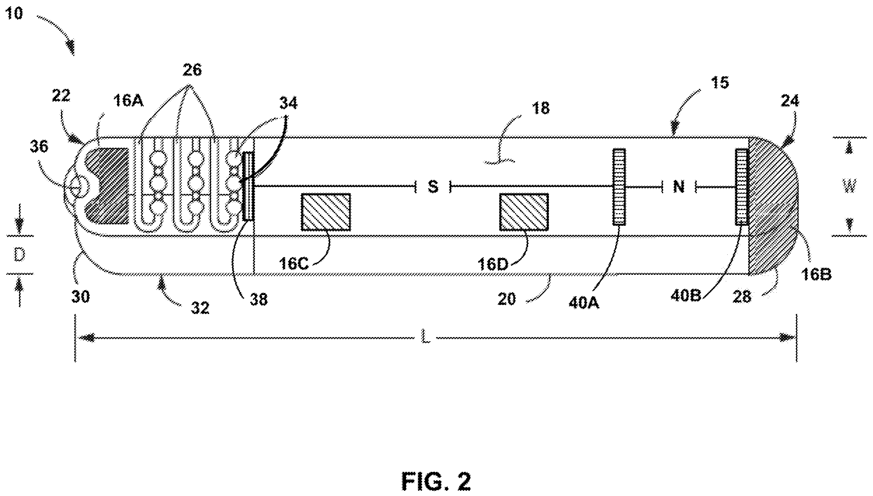

FIG. 2 is a conceptual drawing illustrating an example configuration of IMD 10 of the medical device system 2 of FIG. 1, in accordance with one or more techniques described herein. In the example shown in FIG. 2, IMD 10 may include a leadless, subcutaneously-implantable monitoring device having housing 15, proximal electrode 16A, and distal electrode 16B. Housing 15 may further include first major surface 18, second major surface 20, proximal end 22, and distal end 24. In some examples, IMD 10 may include one or more additional electrodes 16C, 16D positioned on one or both of major surfaces 18, 20 of IMD 10. Housing 15 encloses electronic circuitry located inside the IMD 10, and protects the circuitry contained therein from fluids such as body fluids. In some examples, electrical feedthroughs provide electrical connection of electrodes 16A-16D, and antenna 26, to circuitry within housing 15. In some examples, electrode 16B may be formed from an uninsulated portion of conductive housing 15.

In the example shown in FIG. 2, IMD 10 is defined by a length L, a width W, and thickness or depth D. In this example, IMD 10 is in the form of an elongated rectangular prism in which length L is significantly greater than width W, and in which width W is greater than depth D. However, other configurations of IMD 10 are contemplated, such as those in which the relative proportions of length L, width W, and depth D vary from those described and shown in FIG. 2. In some examples, the geometry of the IMD 10, such as the width W being greater than the depth D, may be selected to allow IMD 10 to be inserted under the skin of the patient using a minimally invasive procedure and to remain in the desired orientation during insertion. In addition, IMD 10 may include radial asymmetries (e.g., the rectangular shape) along a longitudinal axis of IMD 10, which may help maintain the device in a desired orientation following implantation.

In some examples, a spacing between proximal electrode 16A and distal electrode 16B may range from about 30-55 mm, about 35-55 mm, or about 40-55 mm, or more generally from about 25-60 mm. Overall, IMD 10 may have a length L of about 20-30 mm, about 40-60 mm, or about 45-60 mm. In some examples, the width W of major surface 18 may range from about 3-10 mm, and may be any single width or range of widths between about 3-10 mm. In some examples, a depth D of IMD 10 may range from about 2-9 mm. In other examples, the depth D of IMD 10 may range from about 2-5 mm, and may be any single or range of depths from about 2-9 mm. In any such examples, IMD 10 is sufficiently compact to be implanted within the subcutaneous space of patient 4 in the region of a pectoral muscle.

IMD 10, according to an example of the present disclosure, may have a geometry and size designed for ease of implant and patient comfort. Examples of IMD 10 described in this disclosure may have a volume of 3 cubic centimeters (cm³) or less, 1.5 cm³ or less, or any volume therebetween. In addition, in the example shown in FIG. 2, proximal end 22 and distal end 24 are rounded to reduce discomfort and irritation to surrounding tissue once implanted under the skin of patient 4. In some examples, a configuration of IMD 10, including instrument and method for inserting IMD 10 is described, for example, in U.S. Patent Publication No. 2014/0276928. In some examples, a configuration of IMD 10 is described, for example, in U.S. Patent Publication No. 2016/0310031.

In the example shown in FIG. 2, first major surface 18 of IMD 10 faces outward towards the skin, when IMD 10 is inserted within patient 4, whereas second major surface 20 is faces inward toward musculature of patient 4. Thus, first and second major surfaces 18, 20 may face in directions along a sagittal axis of patient 4 (see FIG. 1), and this orientation may be maintained upon implantation due to the dimensions of IMD 10.

Proximal electrode 16A and distal electrode 16B may be used to sense cardiac EGM signals (e.g., ECG signals) when IMD 10 is implanted subcutaneously in patient 4. In some examples, processing circuitry of IMD 10 also may determine whether cardiac ECG signals of patient 4 are indicative of arrhythmia or other abnormalities, which processing circuitry of IMD 10 may evaluate in determining whether a medical condition (e.g., heart failure, sleep apnea, or COPD) of patient 4 has changed. The cardiac ECG signals may be stored in a memory of the IMD 10, and data derived from the cardiac ECG signals may be transmitted via integrated antenna 26 to another medical device, such as external device 12. In some examples, one or both of electrodes 16A and 16B also may be used by IMD 10 to detect impedance values during impedance measurements performed by IMD 10. In some examples, such impedance values detected by IMD 10 may reflect a resistance value associated with a contact between electrodes 16A, 16B, and target tissue of patient 4. Additionally, in some examples, electrodes 16A, 16B may be used by communication circuitry of IMD 10 for tissue conductance communication (TCC) communication with external device 12 or another device.

In the example shown in FIG. 2, proximal electrode 16A is in close proximity to proximal end 22, and distal electrode 16B is in close proximity to distal end 24 of IMD 10. In this example, distal electrode 16B is not limited to a flattened, outward facing surface, but may extend from first major surface 18, around rounded edges 28 or end surface 30, and onto the second major surface 20 in a three-dimensional curved configuration. As illustrated, proximal electrode 16A is located on first major surface 18 and is substantially flat and outward facing. However, in other examples not shown here, proximal electrode 16A and distal electrode 16B both may be configured like proximal electrode 16A shown in FIG. 2, or both may be configured like distal electrode 16B shown in FIG. 2. In some examples, additional electrodes 16C and 16D may be positioned on one or both of first major surface 18 and second major surface 20, such that a total of four electrodes are included on IMD 10. Any of electrodes 16A-16D may be formed of a biocompatible conductive material. For example, any of electrodes 16A-16D may be formed from any of stainless steel, titanium, platinum, iridium, or alloys thereof. In addition, electrodes of IMD 10 may be coated with a material such as titanium nitride or fractal titanium nitride, although other suitable materials and coatings for such electrodes may be used.

In the example shown in FIG. 2, proximal end 22 of IMD 10 includes header assembly 32 having one or more of proximal electrode 16A, integrated antenna 26, anti-migration projections 34, and suture hole 36. Integrated antenna 26 is located on the same major surface (e.g., first major surface 18) as proximal electrode 16A, and may be an integral part of header assembly 32. In other examples, integrated antenna 26 may be formed on the major surface opposite from proximal electrode 16A, or, in still other examples, may be incorporated within housing 15 of IMD 10. Antenna 26 may be configured to transmit or receive electromagnetic signals for communication. For example, antenna 26 may be configured to transmit to or receive signals from a programmer via inductive coupling, electromagnetic coupling, tissue conductance, Near Field Communication (NFC), Radio Frequency Identification (RFID), Bluetooth^{®}, WiFi^{®}, or other proprietary or non-proprietary wireless telemetry communication schemes. Antenna 26 may be coupled to communication circuitry of IMD 10, which may drive antenna 26 to transmit signals to external device 12, and may transmit signals received from external device 12 to processing circuitry of IMD 10 via communication circuitry.

IMD 10 may include several features for retaining IMD 10 in position once subcutaneously implanted in patient 4. For example, as shown in FIG. 2, housing 15 may include anti-migration projections 34 positioned adjacent integrated antenna 26. Anti-migration projections 34 may include a plurality of bumps or protrusions extending away from first major surface 18, and may help prevent longitudinal movement of IMD 10 after implantation in patient 4. In other examples, anti-migration projections 34 may be located on the opposite major surface as proximal electrode 16A and/or integrated antenna 26. In addition, in the example shown in FIG. 2 header assembly 32 includes suture hole 36, which provides another means of securing IMD 10 to the patient to prevent movement following insertion. In the example shown, suture hole 36 is located adjacent to proximal electrode 16A. In some examples, header assembly 32 may include a molded header assembly made from a polymeric or plastic material, which may be integrated or separable from the main portion of IMD 10.

Electrodes 16A and 16B may be used to sense cardiac ECG signals, as described above. Additional electrodes 16C and 16D may be used to sense subcutaneous tissue impedance, in addition to or instead of electrodes 16A, 16B, in some examples. In some examples, processing circuitry of IMD 10 may determine an impedance value of patient 4 based on signals received from at least two of electrodes 16A-16D. For example, processing circuitry of IMD 10 may generate one of a current or voltage signal, deliver the signal via a selected two or more of electrodes 16A-16D, and measure the resulting other of current or voltage. Processing circuitry of IMD 10 may determine an impedance value based on the delivered current or voltage and the measured voltage or current.

In the example shown in FIG. 2, IMD 10 includes a light emitter 38, a proximal light detector 40A, and a distal light detector 40B positioned on housing 15 of IMD 10. Light detector 40A may be positioned at a distance S from light emitter 38, and a distal light detector 40B positioned at a distance S+N from light emitter 38. In other examples, IMD 10 may include only one of light detectors 40A, 40B, or may include additional light emitters and/or additional light detectors. Although light emitter 38 and light detectors 40A, 40B are described herein as being positioned on housing 15 of IMD 10, in other examples, one or more of light emitter 38 and light detectors 40A, 40B may be positioned, on a housing of another type of IMD within patient 4, such as a transvenous, subcutaneous, or extravascular pacemaker or ICD, or connected to such a device via a lead.

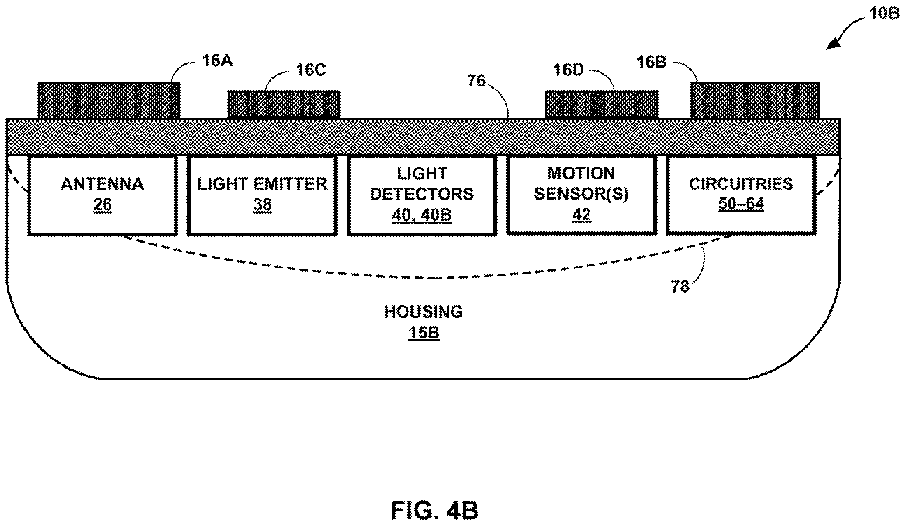

As shown in FIG. 2, light emitter 38 may be positioned on header assembly 32, although, in other examples, one or both of light detectors 40A, 40B may additionally or alternatively be positioned on header assembly 32. In some examples, light emitter 38 may be positioned on a medial section of IMD 10, such as part way between proximal end 22 and distal end 24. Although light emitter 38 and light detectors 40A, 40B are illustrated as being positioned on first major surface 18, light emitter 38 and light detectors 40A, 40B alternatively may be positioned on second major surface 20. In some examples, IMD may be implanted such that light emitter 38 and light detectors 40A, 40B face inward when IMD 10 is implanted, toward the muscle of patient 4, which may help minimize interference from background light coming from outside the body of patient 4. Light detectors 40A, 40B may include a glass or sapphire window, such as described below with respect to FIG. 4B, or may be positioned beneath a portion of housing 15 of IMD 10 that is made of glass or sapphire, or otherwise transparent or translucent.

In some examples, IMD 10 may include one or more additional sensors, such as one or more accelerometers (not shown). Such accelerometers may be 3D accelerometers configured to generate signals indicative of one or more types of movement of the patient, such as gross body movement (e.g., motion) of the patient, patient posture, movements associated with the beating of the heart, or coughing, rales, or other respiration abnormalities. One or more of the parameters monitored by IMD 10 (e.g., impedance, EGM) may fluctuate in response to changes in one or more such types of movement. For example, changes in parameter values sometimes may be attributable to increased patient motion (e.g., exercise or other physical motion as compared to immobility) or to changes in patient posture, and not necessarily to changes in a medical condition. Thus, in some methods of identifying or tracking a medical condition of patient 4, it may be advantageous to account for such fluctuations when determining whether a change in a parameter is indicative of a change in a medical condition.

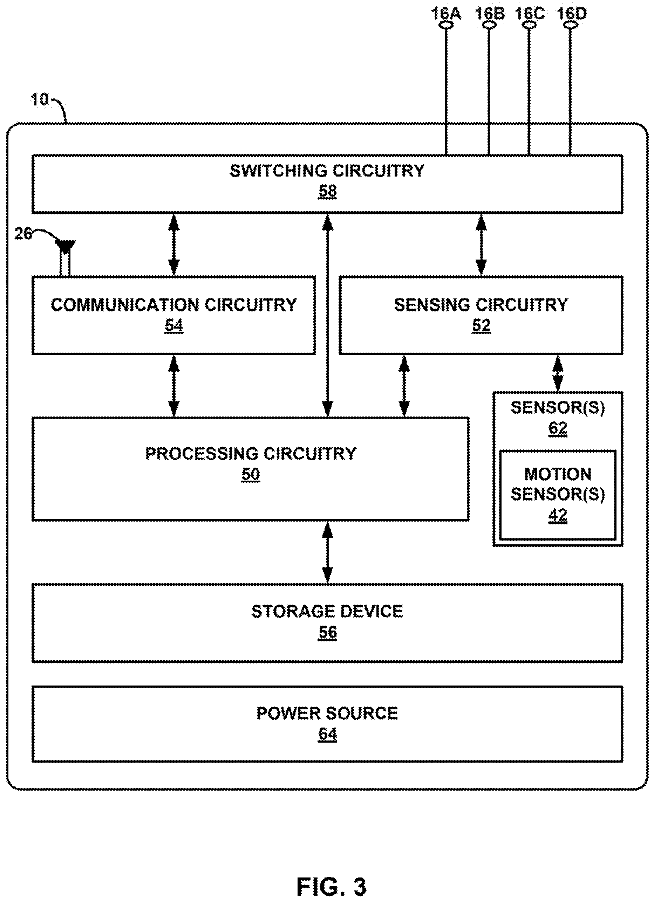

FIG. 3 is a functional block diagram illustrating an example configuration of IMD 10 of FIGS. 1 and 2, in accordance with one or more techniques described herein. In the illustrated example, IMD 10 includes electrodes 16, antenna 26, processing circuitry 50, sensing circuitry 52, communication circuitry 54, storage device 56, switching circuitry 58, sensors 62 including motion sensor(s) 42, and power source 64. Although not illustrated in FIG. 3, Sensors 62 may include light detectors 40 of FIG. 2.

Processing circuitry 50 may include fixed function circuitry and/or programmable processing circuitry. Processing circuitry 50 may include any one or more of a microprocessor, a controller, a DSP, an ASIC, an FPGA, or equivalent discrete or analog logic circuitry. In some examples, processing circuitry 50 may include multiple components, such as any combination of one or more microprocessors, one or more controllers, one or more DSPs, one or more ASICs, or one or more FPGAs, as well as other discrete or integrated logic circuitry. The functions attributed to processing circuitry 50 herein may be embodied as software, firmware, hardware or any combination thereof.

Sensing circuitry 52 and communication circuitry 54 may be selectively coupled to electrodes 16A-16D via switching circuitry 58, as controlled by processing circuitry 50. Sensing circuitry 52 may monitor signals from electrodes 16A-16D in order to monitor electrical activity of heart (e.g., to produce an ECG), and/or subcutaneous tissue impedance, the impedance being indicative of at least some aspects of patient 4's respiratory patterns. Sensing circuitry 52 also may monitor signals from sensors 62, which may include motion sensor(s) 42, and any additional light detectors that may be positioned on IMD 10. In some examples, sensing circuitry 52 may include one or more filters and amplifiers for filtering and amplifying signals received from one or more of electrodes 16A-16D and/or motion sensor(s) 42.

Communication circuitry 54 may include any suitable hardware, firmware, software or any combination thereof for communicating with another device, such as external device 12 or another IMD or sensor, such as a pressure sensing device. Under the control of processing circuitry 50, communication circuitry 54 may receive downlink telemetry from, as well as send uplink telemetry to, external device 12 or another device with the aid of an internal or external antenna, e.g., antenna 26. In addition, processing circuitry 50 may communicate with a networked computing device via an external device (e.g., external device 12) and a computer network, such as the Medtronic CareLink^{®} Network developed by Medtronic, plc, of Dublin, Ireland.

A clinician or other user may retrieve data from IMD 10 using external device 12, or by using another local or networked computing device configured to communicate with processing circuitry 50 via communication circuitry 54. The clinician may also program parameters of IMD 10 using external device 12 or another local or networked computing device.

In some examples, storage device 56 includes computer-readable instructions that, when executed by processing circuitry 50, cause IMD 10 and processing circuitry 50 to perform various functions attributed to IMD 10 and processing circuitry 50 herein. Storage device 56 may include any volatile, non-volatile, magnetic, optical, or electrical media, such as a random access memory (RAM), read-only memory (ROM), non-volatile RAM (NVRAM), electrically-erasable programmable ROM (EEPROM), flash memory, or any other digital media.

Power source 64 is configured to deliver operating power to the components of IMD 10. Power source 64 may include a battery and a power generation circuit to produce the operating power. In some examples, the battery is rechargeable to allow extended operation. In some examples, recharging is accomplished through proximal inductive interaction between an external charger and an inductive charging coil within external device 12. Power source 64 may include any one or more of a plurality of different battery types, such as nickel cadmium batteries and lithium ion batteries. A non-rechargeable battery may be selected to last for several years, while a rechargeable battery may be inductively charged from an external device, e.g., on a daily or weekly basis.

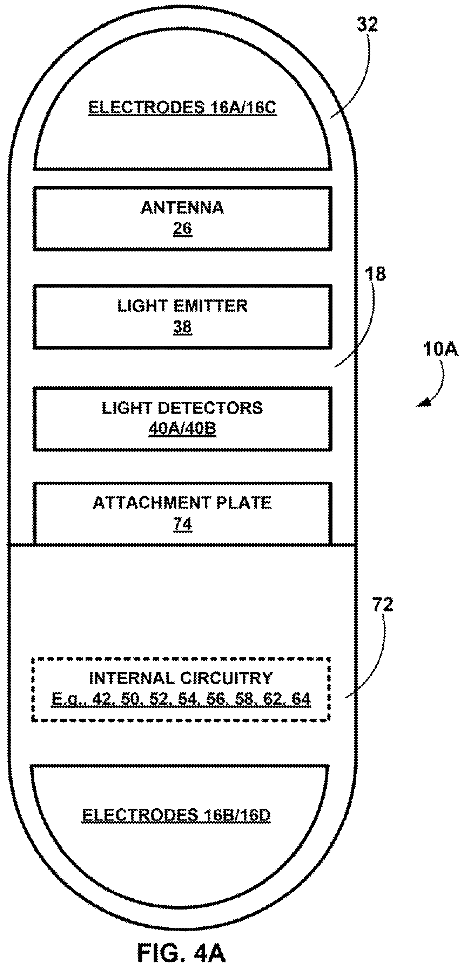

FIGS. 4A and 4B illustrate two additional example IMDs that may be substantially similar to IMD 10 of FIGS. 1-3, but which may include one or more additional features, in accordance with one or more techniques described herein. The components of FIGS. 4A and 4B may not necessarily be drawn to scale, but instead may be enlarged to show detail. FIG. 4A is a block diagram of a top view of an example configuration of an IMD 10A. FIG. 4B is a block diagram of a side view of example IMD 10B, which may include an insulative layer as described below.

FIG. 4A is a conceptual drawing illustrating another example IMD 10A that may be substantially similar to IMD 10 of FIG. 1. In addition to the components illustrated in FIGS. 1-3, the example of IMD 10 illustrated in FIG. 4A also may include a body portion 72 and an attachment plate 74. Attachment plate 74 may be configured to mechanically couple header assembly 32 to body portion 72 of IMD 10A. Body portion 72 of IMD 10A may be configured to house one or more of the internal components of IMD 10 illustrated in FIG. 3, such as one or more of processing circuitry 50, sensing circuitry 52, communication circuitry 54, storage device 56, switching circuitry 58, internal components of sensors 62, and power source 64. In some examples, body portion 72 may be formed of one or more of titanium, ceramic, or any other suitable biocompatible materials.

FIG. 4B is a conceptual drawing illustrating another example IMD 10B that may include components substantially similar to IMD 10 of FIG. 1. In addition to the components illustrated in FIGS. 1-3, the example of IMD 10B illustrated in FIG. 4B also may include a wafer-scale insulative cover 76, which may help insulate electrical signals passing between electrodes 16A-16D and/or light detectors 40A, 40B on housing 15B and processing circuitry 50. In some examples, insulative cover 76 may be positioned over an open housing 15 to form the housing for the components of IMD 10B. One or more components of IMD 10B (e.g., antenna 26, light emitter 38, light detectors 40A, 40B, processing circuitry 50, sensing circuitry 52, communication circuitry 54, switching circuitry 58, and/or power source 64) may be formed on a bottom side of insulative cover 76, such as by using flip-chip technology. Insulative cover 76 may be flipped onto a housing 15B. When flipped and placed onto housing 15B, the components of IMD 10B formed on the bottom side of insulative cover 76 may be positioned in a gap 78 defined by housing 15B.

Insulative cover 76 may be configured so as not to interfere with the operation of IMD 10B. For example, one or more of electrodes 16A-16D may be formed or placed above or on top of insulative cover 76, and electrically connected to switching circuitry 58 through one or more vias (not shown) formed through insulative cover 76. Insulative cover 76 may be formed of sapphire (i.e., corundum), glass, parylene, and/or any other suitable insulating material. Sapphire may be greater than 80% transmissive for wavelengths in the range of about 300 nm to about 4000 nm, and may have a relatively flat profile. In the case of variation, different transmissions at different wavelengths may be compensated for, such as by using a ratiometric approach. In some examples, insulative cover 76 may have a thickness of about 300 micrometers to about 600 micrometers. Housing 15B may be formed from titanium or any other suitable material (e.g., a biocompatible material), and may have a thickness of about 200 micrometers to about 500 micrometers. These materials and dimensions are examples only, and other materials and other thicknesses are possible for devices of this disclosure.

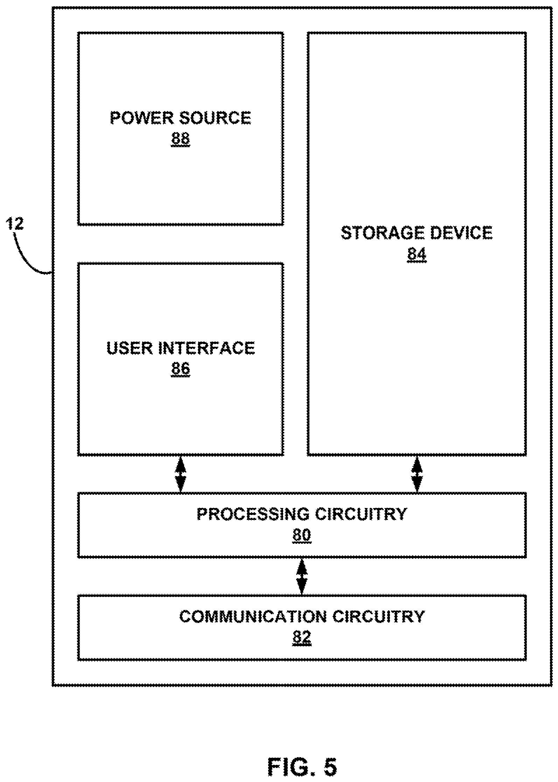

FIG. 5 is a block diagram illustrating an example configuration of components of external device 12, in accordance with one or more techniques of this disclosure. In the example of FIG. 5, external device 12 includes processing circuitry 80, communication circuitry 82, storage device 84, user interface 86, and power source 88.

Processing circuitry 80, in one example, may include one or more processors that are configured to implement functionality and/or process instructions for execution within external device 12. For example, processing circuitry 80 may be capable of processing instructions stored in storage device 84. Processing circuitry 80 may include, for example, microprocessors, DSPs, ASICs, FPGAs, or equivalent discrete or integrated logic circuitry, or a combination of any of the foregoing devices or circuitry. Accordingly, processing circuitry 80 may include any suitable structure, whether in hardware, software, firmware, or any combination thereof, to perform the functions ascribed herein to processing circuitry 80.

Communication circuitry 82 may include any suitable hardware, firmware, software or any combination thereof for communicating with another device, such as IMD 10. Under the control of processing circuitry 80, communication circuitry 82 may receive downlink telemetry from, as well as send uplink telemetry to, IMD 10, or another device.

Storage device 84 may be configured to store information within external device 12 during operation. Storage device 84 may include a computer-readable storage medium or computer-readable storage device. In some examples, storage device 84 includes one or more of a short-term memory or a long-term memory. Storage device 84 may include, for example, RAM, dynamic random access memories (DRAM), static random access memories (SRAM), magnetic discs, optical discs, flash memories, or forms of electrically programmable memories (EPROM) or EEPROM. In some examples, storage device 84 is used to store data indicative of instructions for execution by processing circuitry 80. Storage device 84 may be used by software or applications running on external device 12 to temporarily store information during program execution.

Data exchanged between external device 12 and IMD 10 may include operational parameters. External device 12 may transmit data including computer readable instructions which, when implemented by IMD 10, may control IMD 10 to change one or more operational parameters and/or export collected data. For example, processing circuitry 80 may transmit an instruction to IMD 10 which requests IMD 10 to export collected data (e.g., data corresponding to one or both of an ECG signal and an accelerometer signal) to external device 12. In turn, external device 12 may receive the collected data from IMD 10 and store the collected data in storage device 84. Additionally, or alternatively, processing circuitry 80 may export instructions to IMD 10 requesting IMD 10 to update electrode combinations for stimulation or sensing.

A user, such as a clinician or patient 4, may interact with external device 12 through user interface 86. User interface 86 includes a display (not shown), such as an LCD or LED display or other type of screen, with which processing circuitry 80 may present information related to IMD 10 (e.g., EGM signals obtained from at least one electrode or at least one electrode combination). In addition, user interface 86 may include an input mechanism to receive input from the user. The input mechanisms may include, for example, any one or more of buttons, a keypad (e.g., an alphanumeric keypad), a peripheral pointing device, a touch screen, or another input mechanism that allows the user to navigate through user interfaces presented by processing circuitry 80 of external device 12 and provide input. In other examples, user interface 86 also includes audio circuitry for providing audible notifications, instructions or other sounds to patient 4, receiving voice commands from patient 4, or both. Storage device 84 may include instructions for operating user interface 86 and for managing power source 88.

Power source 88 is configured to deliver operating power to the components of external device 12. Power source 88 may include a battery and a power generation circuit to produce the operating power. In some examples, the battery is rechargeable to allow extended operation. Recharging may be accomplished by electrically coupling power source 88 to a cradle or plug that is connected to an alternating current (AC) outlet. In addition, recharging may be accomplished through proximal inductive interaction between an external charger and an inductive charging coil within external device 12. In other examples, traditional batteries (e.g., nickel cadmium or lithium ion batteries) may be used. In addition, external device 12 may be directly coupled to an alternating current outlet to operate.

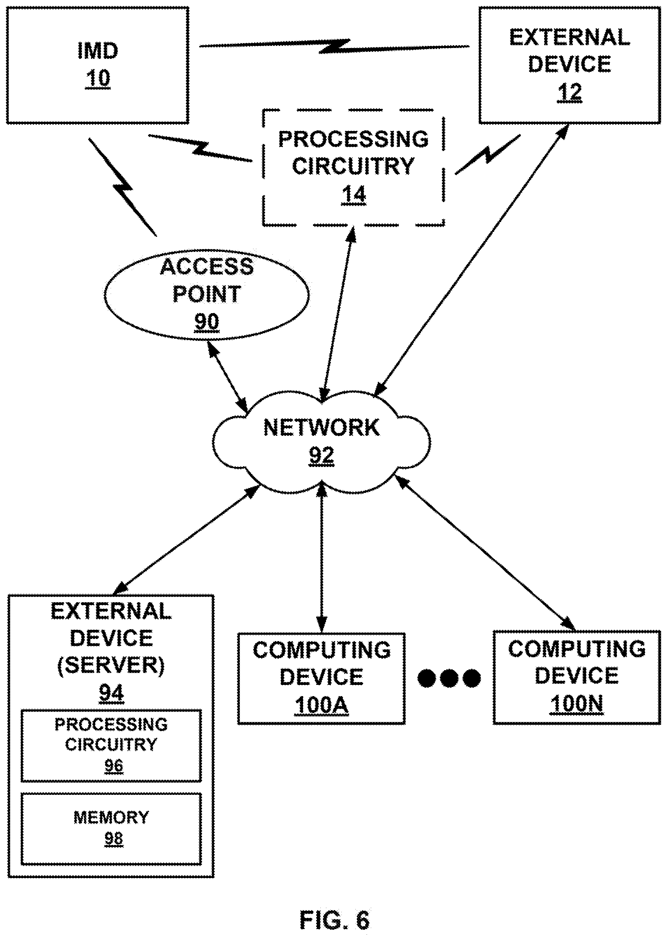

FIG. 6 is a block diagram illustrating an example system that includes an access point 90, a network 92, external computing devices, such as a server 94, and one or more other computing devices 100A-100N, which may be coupled to IMD 10, external device 12, and processing circuitry 14 via network 92, in accordance with one or more techniques described herein. In this example, IMD 10 may use communication circuitry 54 to communicate with external device 12 via a first wireless connection, and to communication with an access point 90 via a second wireless connection. In the example of FIG. 6, access point 90, external device 12, server 94, and computing devices 100A-100N are interconnected and may communicate with each other through network 92.

Access point 90 may include a device that connects to network 92 via any of a variety of connections, such as telephone dial-up, digital subscriber line (DSL), or cable modem connections. In other examples, access point 90 may be coupled to network 92 through different forms of connections, including wired or wireless connections. In some examples, access point 90 may be a user device, such as a tablet or smartphone, that may be co-located with the patient. As discussed above, IMD 10 may be configured to transmit data, such as any one or combination of an EGM signal, an accelerometer signal, and a cough count to external device 12. In addition, access point 90 may interrogate IMD 10, such as periodically or in response to a command from the patient or network 92, in order to retrieve parameter values determined by processing circuitry 50 of IMD 10, or other operational or patient data from IMD 10. Access point 90 may then communicate the retrieved data to server 94 via network 92.

In some cases, server 94 may be configured to provide a secure storage site for data that has been collected from IMD 10, and/or external device 12. In some cases, server 94 may assemble data in web pages or other documents for viewing by trained professionals, such as clinicians, via computing devices 100A-100N. One or more aspects of the illustrated system of FIG. 6 may be implemented with general network technology and functionality, which may be similar to that provided by the Medtronic CareLink^{®} Network developed by Medtronic plc, of Dublin, Ireland.

Server 94 may include processing circuitry 96. Processing circuitry 96 may include fixed function circuitry and/or programmable processing circuitry. Processing circuitry 96 may include any one or more of a microprocessor, a controller, a DSP, an ASIC, an FPGA, or equivalent discrete or analog logic circuitry. In some examples, processing circuitry 96 may include multiple components, such as any combination of one or more microprocessors, one or more controllers, one or more DSPs, one or more ASICs, or one or more FPGAs, as well as other discrete or integrated logic circuitry. The functions attributed to processing circuitry 96 herein may be embodied as software, firmware, hardware or any combination thereof. In some examples, processing circuitry 96 may perform one or more techniques described herein based on an EGM signal and/or an accelerometer signal received from IMD 10, or based on a cough count value received from IMD 10, as examples.

Server 94 may include memory 98. Memory 98 includes computer-readable instructions that, when executed by processing circuitry 96, cause IMD 10 and processing circuitry 96 to perform various functions attributed to IMD 10 and processing circuitry 96 herein. Memory 98 may include any volatile, non-volatile, magnetic, optical, or electrical media, such as RAM, ROM, NVRAM, EEPROM, flash memory, or any other digital media.

In some examples, one or more of computing devices 100A-100N (e.g., device 100A) may be a tablet or other smart device located with a clinician, by which the clinician may program, receive alerts from, and/or interrogate IMD 10. For example, the clinician may access data corresponding to an EGM signal and/or an accelerometer signal collected by IMD 10 through device 100A, such as when patient 4 is in between clinician visits, to check on a status of a medical condition. In some examples, the clinician may enter instructions for a medical intervention for patient 4 into an app in device 100A, such as based on a status of a patient condition determined by IMD 10, external device 12, processing circuitry 14, or any combination thereof, or based on other patient data known to the clinician. Device 100A then may transmit the instructions for medical intervention to another of computing devices 100A-100N (e.g., device 100B) located with patient 4 or a caregiver of patient 4. For example, such instructions for medical intervention may include an instruction to change a drug dosage, timing, or selection, to schedule a visit with the clinician, or to seek medical attention. In further examples, device 100B may generate an alert to patient 4 based on a status of a medical condition of patient 4 determined by IMD 10, which may enable patient 4 proactively to seek medical attention prior to receiving instructions for a medical intervention. In this manner, patient 4 may be empowered to take action, as needed, to address his or her medical status, which may help improve clinical outcomes for patient 4.

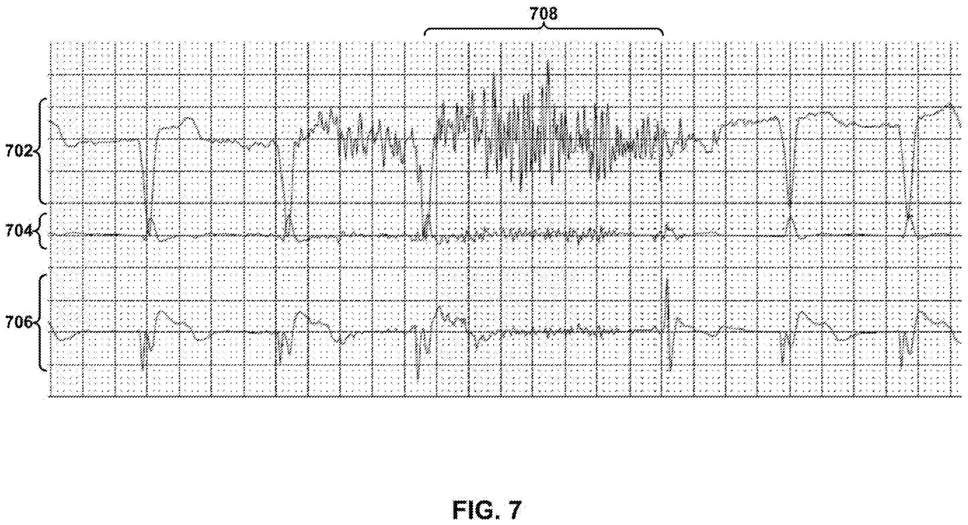

FIG. 7 is a graph illustrating a first EGM plot 702, a second EGM plot 704, and a third EGM plot 706, in accordance with one or more techniques of this disclosure. In some examples, one or more of the first EGM plot 702, the second EGM plot 704, and the third EGM plot 706 are recorded by IMD 10. In some examples, one or more of the first EGM plot 702, the second EGM plot 704, and the third EGM plot 706 are recorded by one or more additional or alternative devices. First EGM plot 702 may represent a first far-field EGM, second EGM plot 704 may represent a second far-field EGM, and third EGM plot 706 may represent a near-field EGM. The terms "far-field" and "near-field" may represent relative distances of electrodes recording an EGM signal from a heart of patient 4. For example, the electrodes which record third EGM plot 706 may be closer to a heart of patient 4 than the electrodes which record first EGM plot 702 and the electrodes which record second EGM plot 704. As seen in FIG. 7, of the first EGM plot 702, the second EGM plot 704, and the third EGM plot 706 include noise during a period of time 708. The noise in the first EGM plot 702, the second EGM plot 704, and the third EGM plot 706 during the period of time 708 may include a higher amplitude than noise in other parts of the first EGM plot 702, the second EGM plot 704, and the third EGM plot 706. In some examples, the noise in the first EGM plot 702, the second EGM plot 704, and the third EGM plot 706 during the period of time 708 may relate to one or more coughs by patient 4. In this way one or more of the first EGM plot 702, the second EGM plot 704, and the third EGM plot 706 may be analyzed by processing circuitry 14 to identify one or more coughs by patient 4.

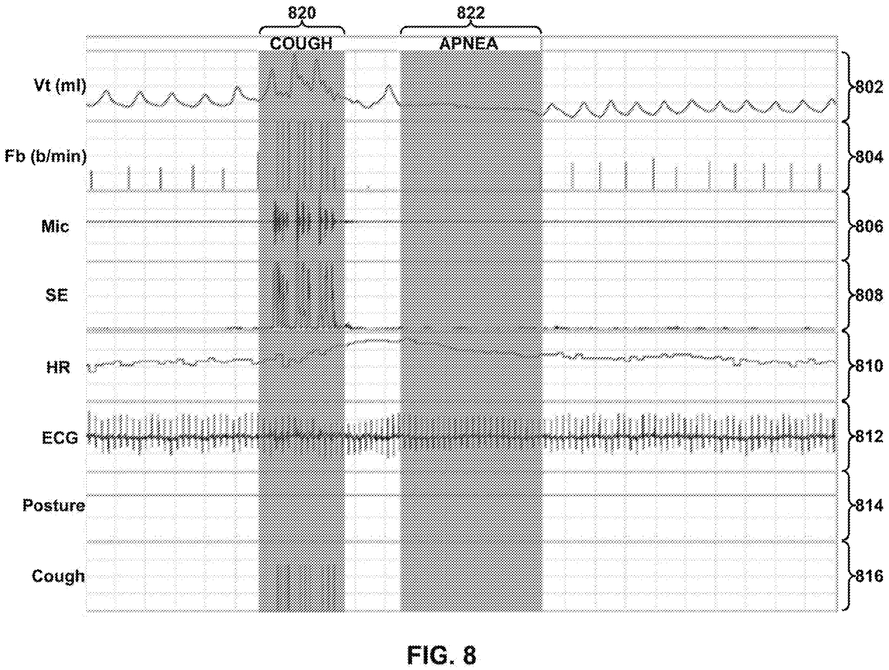

FIG. 8 is a graph illustrating a set of physiological signal plots recorded concurrently, in accordance with one or more techniques of this disclosure. As seen in FIG. 8, the set of physiological signal plots includes a tidal volume (Vt) plot 802, a breathing frequency (Fb) plot 804, a microphone (Mic) signal plot 806, a sound envelope (SE) plot 808, a heart rate (HR) plot 810, an electrocardiogram (ECG) plot 812, a body position plot 814, and a cough indicator plot 816. The physiological signal plots may be recorded during a cough period 820 and an apnea period 822.

As seen in FIG. 8, during the cough period 820, the tidal volume plot 802 indicates an increase in tidal volume, the breathing frequency plot 804 indicates an increase in breathing frequency, the microphone signal plot 806 indicates sounds, an amplitude of the sound envelope plot 808 increases, the heart rate plot 810 indicates an increase in heart rate, baseline noise in the ECG plot 812 increases in amplitude, the body position plot 814 indicates that a posture of the patient remains the same, and the cough indicator plot 816 indicates a detection of several coughs. Additionally, as seen in FIG. 8 during the apnea period 822, the tidal volume plot 802 indicates a decrease in tidal volume, the breathing frequency plot 804 indicates that no breaths occurred during the apnea period 822, the microphone signal plot 806 does not indicate sounds, an amplitude of the sound envelope plot 808 is low as compared with the cough period 820, the heart rate plot 810 indicates a decrease in heart rate, baseline noise in the ECG plot 812 decreases in amplitude, the body position plot 814 indicates that a posture of the patient remains the same, and the cough indicator plot 816 does not indicate a detection of any coughs. In some examples, processing circuitry such as processing circuitry 14 may use one or more physiological signal plots of the set of physiological signal plots illustrated in FIG. 8 to identify one or more coughs by patient 4. Additionally, or alternatively, in some examples, processing circuitry such as processing circuitry 14 may use one or more physiological signal plots of the set of physiological signal plots illustrated in FIG. 8 to identify one or more periods of apnea in patient 4.

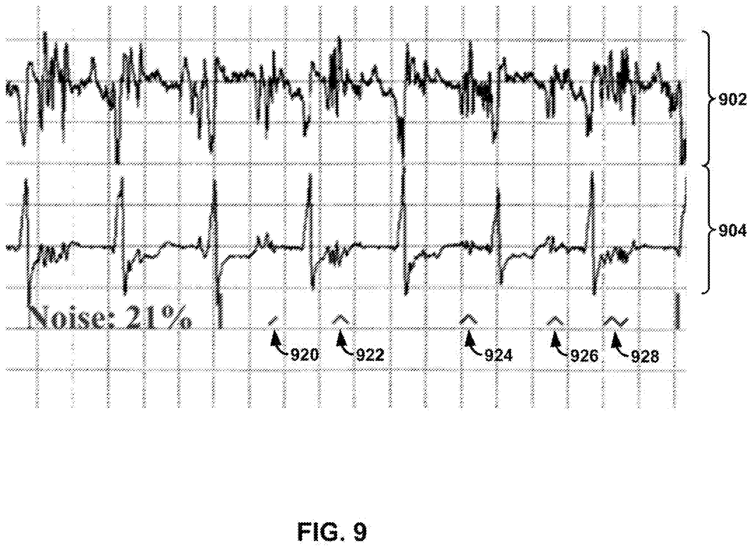

FIG. 9 is a graph illustrating a far-field EGM plot 902 and a near-field EGM plot 904, in accordance with one or more techniques of this disclosure. Additionally, FIG. 9 includes slope reversal indication 920, slope reversal indications 922, slope reversal indications 924, slope reversal indications 926, and slope reversal indications 928. In some examples, processing circuitry 14 may detect one or more slope reversals based on an EGM signal, such as the EGM signals given in one or both of far-field EGM plot 902 and near-field EGM plot 904. In some examples, far-field EGM plot 902 includes a first set of EGM samples and near-field EGM plot 904 includes a second set of EGM samples. Processing circuitry 14 may determine one or more slope reversals in one or both of the first set of EGM samples and the second set of EGM samples based on one or more techniques described herein. Slope reversal indication 920 may indicate one slope reversal. Slope reversal indications 922 may indicate two slope reversals. Slope reversal indications 924 may indicate two slope reversals. Slope reversal indications 926 may indicate two slope reversals. Slope reversal indications 928 may indicate three slope reversals.

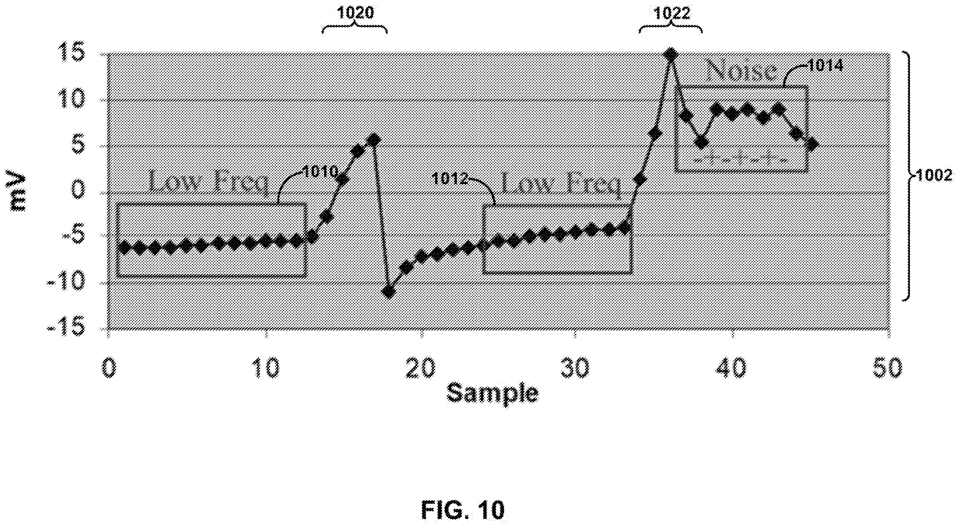

FIG. 10 is a graph illustrating a sequence of EGM samples 1002 representing an EGM signal, in accordance with one or more techniques of this disclosure. As seen in FIG. 10, the sequence of EGM samples 1002 includes a first set of EGM samples 1010, a second set of EGM samples, 1012, and a third set of EGM samples 1014. The first set of EGM samples 1010 and the second set of EGM samples might not include noise relating to one or more coughs by patient 4. The third set of EGM samples 1014, on the other hand, may include noise relating to one or more coughs by patient 4. The sequence of EGM samples 1002 includes first R-wave 1020 and second R-wave 1022 (collectively, "R-waves 1020, 1022"). In some examples, processing circuitry 14 may determine that the third set of EGM samples 1014 includes noise relating to one or more coughs of patient 4 (e.g., noise relating to any one or combination of contractions of the diaphragm, contractions of abdominal muscles, and contractions of intercostal muscles) based on one or techniques described herein.

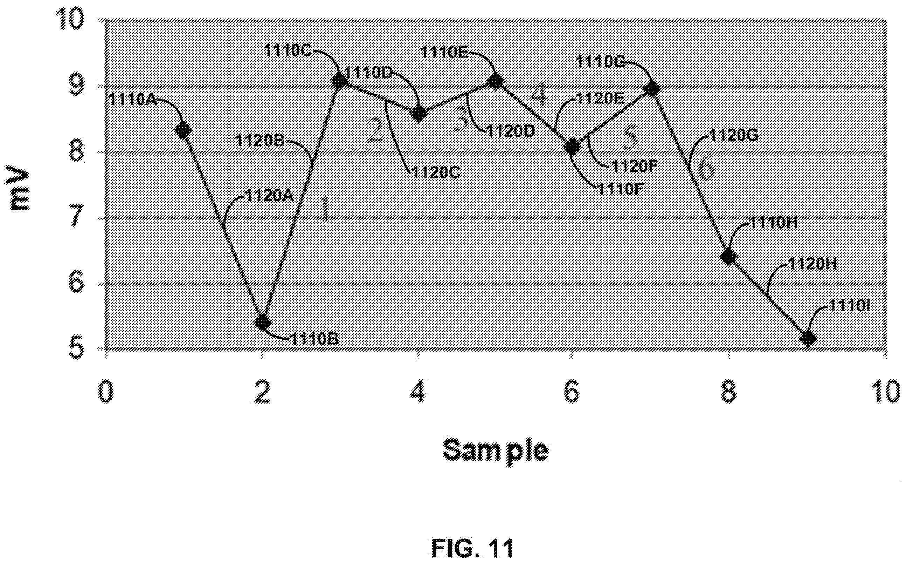

FIG. 11 is a graph illustrating a group of EGM samples 1110A-1110I (collectively, "EGM samples 1110"), in accordance with one or more techniques of this disclosure. EGM samples 1110 may be an example of the third set of EGM samples 1014 of FIG. 10. Additionally, the graph includes lines 1120A-1120H (collectively, "lines 1120") which connect pairs of consecutive EGM samples 1110. As seen in FIG. 11, line 1120A connects EGM sample 1110A and EGM sample 1110B, line 1120B connects EGM sample 1110B and EGM sample 1110C, line 1120C connects EGM sample 1110C and EGM sample 1110D, line 1120D connects EGM sample 1110D and EGM sample 1110E, line 1120E connects EGM sample 1110E and EGM sample 1110F, line 1120F connects EGM sample 1110F and EGM sample 1110G, line 1120G connects EGM sample 1110G and EGM sample 1110H, and line 1120H connects EGM sample 1110H and EGM sample 1110I.

EGM samples 1110 includes a set of slope reversals. For example, line 1120A and line 1120B may represent a slope reversal since line 1120A has a negative slope and line 1120B has a positive slope. As such, a slope change event occurs at EGM sample 1110B. Processing circuitry 14 may determine that a slope change event occurs at EGM sample 1110B by determining that a set of EGM samples representing a derivative of EGM samples 1110 changes sign at a time corresponding to EGM sample 1110B. Additionally, processing circuitry 14 may determine that slope reversals occur at EGM sample 1110C, EGM sample 1110D, EGM sample 1110E, EGM sample 1110F, and EGM sample 1110G.

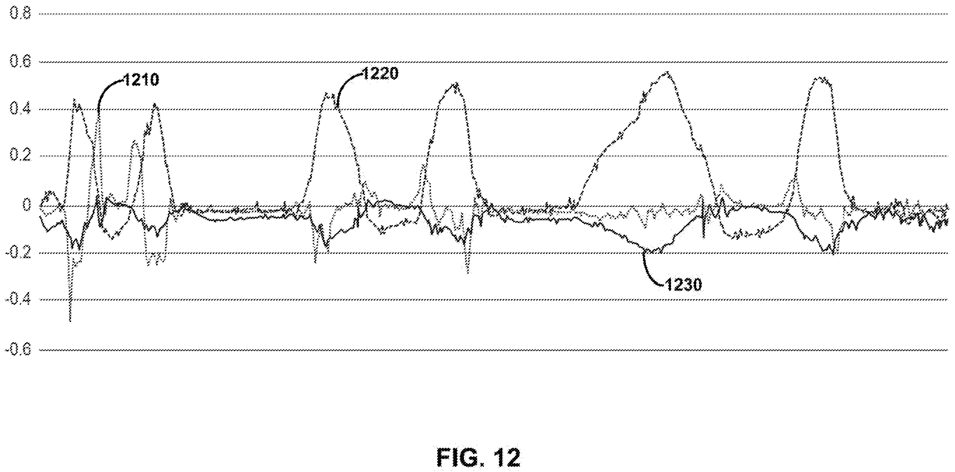

FIG. 12 is a graph illustrating a first accelerometer signal component plot 1210, a second accelerometer signal component plot 1220, and a third accelerometer signal component plot 1230, in accordance with one or more techniques of this disclosure. In some examples, the first accelerometer signal component plot 1210 represents a vertical accelerometer signal component, the second accelerometer signal component plot 1220 represents a frontal accelerometer signal component, and the third accelerometer signal component plot 1230 represents a lateral accelerometer signal component. In some cases, it may be beneficial to analyze second accelerometer signal component plot 1220 to identify one or more coughs of patient 4, since the frontal axis may extend outward form a chest of patient 4 and during a cough, patient 4 may move the chest forward along the frontal axis causing a sharp increase in the frontal accelerometer component. Such sharp increases may register in the frontal accelerometer component and processing circuitry 14 may identify the sharp increases.

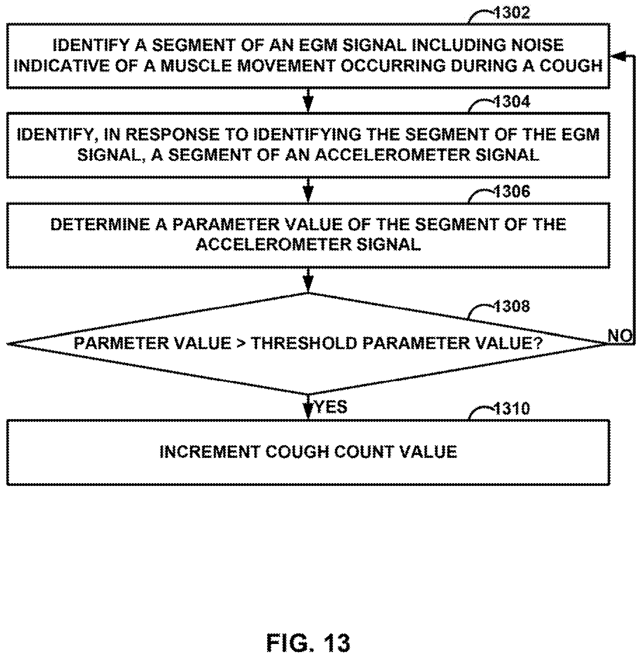

FIG. 13 is a flow diagram illustrating an example operation for detecting one or more coughs of a patient, in accordance with one or more techniques of this disclosure. FIG. 13 is described with respect to IMD 10, external device 12, and processing circuitry 14 of FIGS. 1-6. However, the techniques of FIG. 13 may be performed by different components of IMD 10, external device 12, and processing circuitry 14 or by additional or alternative medical device systems. Processing circuitry 14 is conceptually illustrated in FIG. 1 as separate from IMD 10 and external device 12, but may be processing circuitry of IMD 10 and/or processing circuitry of external device 12. In general, the techniques of this disclosure may be performed by processing circuitry 14 of one or more devices of a system, such as one or more devices that include sensors that provide signals, or processing circuitry of one or more devices that do not include sensors, but nevertheless analyze signals using the techniques described herein. For example, another external device (not pictured in FIG. 1) may include at least a portion of processing circuitry 14, the other external device configured for remote communication with IMD 10 and/or external device 12 via a network.

IMD 10 may collect an EGM signal and an accelerometer signal. In some cases, IMD 10 may collect at least a portion of the EGM signal over a same period of time that IMD 10 collects at least a portion of the accelerometer signal. In this way, the EGM signal and the accelerometer signal may overlap for at least a portion of time. Processing circuitry 14 may detect one or more coughs based on the EGM signal and the accelerometer signal collected by IMD 10. In some examples, processing circuitry 14 may save one or more portions of the EGM signal and the accelerometer signal to a memory for further analysis based on an analysis of the EGM signal.

Processing circuitry 14 identifies a segment of an EGM signal including noise indicative of a muscle movement occurring during a cough (1302). For example, processing circuitry 14 may identify the segment of the EGM signal as a section of noise relating to a contraction of one or more muscles that cause patient 4 to cough. IMD 10 may be implanted near at least some muscles of patient 4 which may contribute to a cough (e.g., diaphragm and intercostal muscles). As such, the EGM signal recorded by IMD 10 may include muscle signals corresponding to a cough by patient 4. For example, IMD 10 may detect the muscle noise in the EGM signal due to contractions in the muscles contributing to a cough, where the muscle noise does not necessarily originate from the heart of patient 4. In this way, processing circuitry 14 may analyze the EGM signal recorded by IMD 10 in order to identify sections of the EGM signal which include the muscle noise that indicates a contraction of muscles due to a cough. In this way, the EGM signal may indicate one or more occurrences of a cough performed by patient 4. Processing circuitry 14 may be configured to detect sections of the EGM signal that include muscle noise relating to a cough according to one or more techniques described herein.

Processing circuitry 14 identifies, in response to identifying the segment of the EGM signal, a segment of an accelerometer signal (1304). In some examples, processing circuitry may identify a period of time in which the segment of the EGM signal is collected by IMD 10. Based on the period of time, processing circuitry 14 may identify the segment of the accelerometer signal. In some examples, processing circuitry 14 may identify the segment of the accelerometer signal as a segment which is collected by IMD 10 over substantially the same period of time in which IMD 10 collects the segment of the EGM signal. In some examples, processing circuitry 14 may identify the segment of the accelerometer signal as a segment which is collected by IMD 10 during a period of time which at least partially overlaps with the period of time in which IMD 10 collects the segment of the EGM signal. In any case, processing circuitry 14 may be configured to analyze both of the accelerometer signal and the EGM signal over a window of time in which processing circuitry 14 identifies a possibility of one or more coughs occurring. More specifically, processing circuitry 14 may analyze the accelerometer signal in response to determining that a segment of the EGM signal may indicate one or more coughs. If the accelerometer signal confirms a cough, processing circuitry 14 may determine an occurrence of a cough.

Processing circuitry 14 determines that a parameter value of the segment of the accelerometer signal (1306). In some examples, the accelerometer signal includes a vertical component, a lateral component, and a frontal component corresponding to a vertical axis, a lateral axis, and a frontal axis, respectively. In this way, the accelerometer signal represents a three-dimensional measurement of acceleration. The vertical axis, the lateral axis, and the frontal axis may represent three axes of a Cartesian space. In this way, the accelerometer signal may track movements of patient 4 within a three-dimensional space. It may be beneficial for processing circuitry 14 to analyze the frontal component of the accelerometer signal in order to determine whether the patient coughed during the period of time in which the segment of the accelerometer signal is being collected. For example, the vertical axis of the accelerometer signal may extend along a torso of patient 4 roughly along a spine of patient 4. The lateral axis may extend, perpendicular to the vertical axis, across a chest of patient 4. Additionally, the frontal axis may extend outwards from the chest of patient 4 perpendicular to the vertical axis and perpendicular to the lateral axis. During a cough, the chest of patient 4 may move forwards along the frontal axis. In this way, the chest movement which occurs due to a cough may be recorded in the frontal component of the accelerometer signal. In this way, the parameter value of the segment of the accelerometer signal may correspond to a magnitude value of the frontal component of the accelerometer signal. Additionally, or alternatively, in some examples, the parameter value may represent one or more of a derivative of the frontal component of the segment of the accelerometer signal, an area under the frontal component of the accelerometer signal, a maximum value of the frontal component within a period of time, a difference between a maximum value of the frontal component and a minimum value of the frontal component over a period of time, a slope associated with a maximum value of the frontal component, or another parameter corresponding to the frontal component, the vertical component, or the lateral component of the accelerometer signal.

Processing circuitry 14 may determine whether the parameter value of the segment of the accelerometer signal is greater than a threshold parameter value (1308). If the parameter value of the segment of the accelerometer signal is not greater than the threshold parameter value ("NO" branch of block 1308), the example operation may return to block 1302 and processing circuitry 14 may identify another segment of the EGM signal including noise indicative of a muscle movement occurring during a cough). In this way, processing circuitry 14 may determine that a cough did not occur during the period of time in which IMD 10 collects the segment of the accelerometer signal if the parameter value is not greater than the threshold parameter value. If the parameter value of the segment of the accelerometer signal is greater than the threshold parameter value ("YES" branch of block 1308), processing circuitry 14 may increment a cough count value (1310). As such, processing circuitry 14 may determine that a cough occurred during the period of time in which IMD 10 collects the segment of the accelerometer signal if the parameter value is greater than the threshold parameter value.

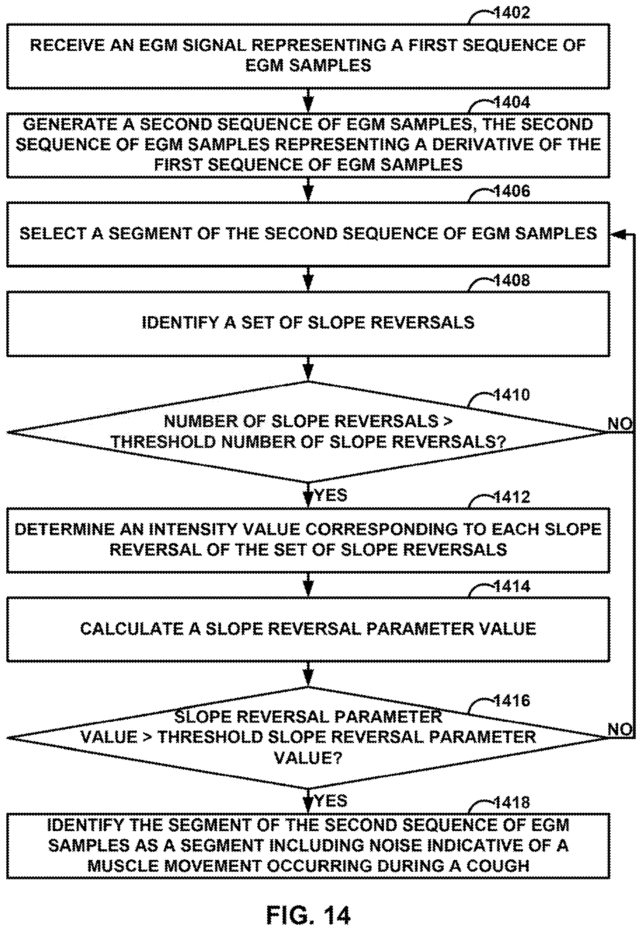

FIG. 14 is a flow diagram illustrating an example operation for identifying a segment of an EGM signal which includes noise that may be indicative of one or more coughs, in accordance with one or more techniques of this disclosure. FIG. 14 is described with respect to IMD 10, external device 12, and processing circuitry 14 of FIGS. 1-6. However, the techniques of FIG. 14 may be performed by different components of IMD 10, external device 12, and processing circuitry 14 or by additional or alternative medical device systems. The example operation of FIG. 14 may represent an example operation for performing block 1302 of FIG. 13 "IDENTIFY A SEGMENT OF AN EGM SIGNAL INCLUDING NOISE INDICATIVE OF A MUSCLE MOVEMENT OCCURRING DURING A COUGH."

Processing circuitry 14 may receive an EGM signal representing a first sequence of EGM samples (1402). In some examples, processing circuitry 14 receives the EGM signal from IMD 10. Subsequently, processing circuitry 14 may generate a second sequence of EGM samples, the second sequence of EGM samples representing a derivative of the first sequence of EGM samples (1404). Processing circuitry 14 may select a segment of the second sequence of second sequence of EGM samples (1406) for cough-related noise analysis. In some examples, processing circuitry 14 may analyze one-second segments of the second sequence of EGM samples in real-time or near real-time according to a sliding one second window. For example, processing circuitry 14 may break the second sequence of EGM samples into one second segments and analyze each segment to determine whether the respective segment includes noise indicative of a cough.

Processing circuitry 14 may identify a set of slope reversals (1408) in a section of the EGM signal corresponding to the segment of the second sequence of EGM samples selected by processing circuitry 14. A slope reversal may be an example of a slope change event. A slope change event may represent any event in which a slope of the EGM signal changes sign (e.g., changes from positive to negative or changes from negative to positive) from a first pair of consecutive EGM samples to a second pair of consecutive EGM samples immediately following the first pair of consecutive EGM samples. However, at least some slope change events might not represent slope reversals. For example, a slope change event may include an R-wave (e.g., a ventricular depolarization). A ventricular depolarization may cause a significant change in the EGM signal and trigger a slope change event. However, a magnitude associated with the slope change event associated with the R-wave may be relatively large compared with other slope change events. As such, processing circuitry 14 may determine that the R-wave is not a slope reversal, and therefore not related to muscle contractions relating to a cough.

One way in which processing circuitry 14 may identify the set of slope reversals in the EGM signal is to identify one or more zero crossings in the segment of the second sequence of EGM samples. A zero crossing may represent a point of zero slope (e.g., a peak or a valley) in the EGM signal collected by IMD 10. Since the second sequence of EGM samples represents a derivative of the first sequence of EGM samples, a zero crossing in the second sequence of EGM samples may represent a slope change event in the first sequence of EGM samples, a slope change event being a point at which a slope of the first sequence of EGM samples changes from positive to negative or negative to positive. Processing circuitry 14, in some cases, may determine a slope change event in the first sequence of EGM samples to be an event in which a pair of consecutive EGM samples of the second sequence of EGM samples changes sign. For example, if an amplitude of a first EGM sample of the pair of consecutive EGM samples is positive and a second EGM sample of the pair of consecutive EGM samples is negative, the pair of consecutive EGM samples may represent a slope change event. Additionally, or alternatively, if the first EGM sample is positive and the second EGM sample is negative, the pair of consecutive EGM samples may represent a slope change event.

In some cases, processing circuitry 14 might not classify at least some slope change events as slope reversals. For example, slope change events that are outside of a range of intensity values might not be classified as slope reversals. In order to determine whether a slope change event is a slope reversal, processing circuitry 14 may calculate an intensity value corresponding to each slope change event detected by processing circuitry 14. The intensity value, in some cases, may be a difference between an amplitude of a first EGM sample and an amplitude of a second EGM sample, where the first EGM sample and the second EGM sample represent a pair of consecutive EGM samples of the second sequence of EGM samples that processing circuitry 14 identifies as a slope change event in the EGM signal collected by IMD 10. If the intensity value of the slope change event is within a range from a first threshold intensity value to a second threshold intensity value, processing circuitry 14 may determine that the slope change event is a slope reversal that is potentially related to a cough of patient 4. On the other hand, if the intensity value of the slope change event is outside of the range from the first threshold intensity value to the second threshold intensity value, processing circuitry 14 may determine that the slope change event is not a slope reversal, and thus not related to a cough of patient 4. For example, processing circuitry 14 may identify an R-wave as a slope change event. However, an intensity value associated with the R-wave may be greater than the second threshold intensity value and processing circuitry 14 may determine that the R-wave does not represent a slope reversal and thus is not related to noise arising from a cough by patient 4.

Processing circuitry 14 may determine whether a number of slope reversals identified in the segment of the second sequence of EGM samples is greater than a threshold number of slope reversals (1410). If the number of slope reversals is not greater than the threshold number of slope reversals ("NO" branch of block 1410), the example operation may return to block 1406 and processing circuitry 14 may select another segment of the second sequence of EGM samples for cough-related noise analysis. If the number of slope reversals is greater than the threshold number of slope reversals ("YES" branch of block 1410), processing circuitry 14 may determine an intensity value corresponding to each slope reversal of the set of slope reversals (1412) identified in the segment of the second sequence of EGM samples. Each slope reversal of the set of slope reversals may be represented by an adjoining pair of consecutive EGM samples, the adjoining pair of consecutive samples including a first EGM sample, a second EGM sample, and a third EGM sample.

After calculating the magnitude corresponding to each slope reversal of the set of slope reversals, processing circuitry 14 may calculate a slope reversal parameter value (1414) corresponding to the set of slope reversals. In some examples, the slope reversal parameter value represents a median magnitude of the set of slope reversals. In some examples, the slope reversal parameter represents a sum of the intensity values corresponding to each slope reversal of the set of slope reversals. Processing circuitry 14 may determine whether a slope reversal parameter value is greater than a threshold slope reversal parameter value (1416). If processing circuitry 14 determines the slope reversal parameter value is not greater than the threshold slope reversal parameter value ("NO" branch of block 1416), the example operation returns to block 1406 and processing circuitry 14 selects another segment of the second sequence of EGM samples. If processing circuitry 14 determines the slope reversal parameter value is greater than the threshold slope reversal parameter value ("YES" branch of block 1416), processing circuitry 14 identifies the segment of the sequence of EGM samples as a segment including noise indicative of a muscle movement occurring during a cough (1418).

The techniques described in this disclosure may be implemented, at least in part, in hardware, software, firmware, or any combination thereof. For example, various aspects of the techniques may be implemented within one or more microprocessors, DSPs, ASICs, FPGAs, or any other equivalent integrated or discrete logic QRS circuitry, as well as any combinations of such components, embodied in external devices, such as physician or patient programmers, stimulators, or other devices. The terms "processor" and "processing circuitry" may generally refer to any of the foregoing logic circuitry, alone or in combination with other logic circuitry, or any other equivalent circuitry, and alone or in combination with other digital or analog circuitry.

For aspects implemented in software, at least some of the functionality ascribed to the systems and devices described in this disclosure may be embodied as instructions on a computer-readable storage medium such as RAM, DRAM, SRAM, magnetic discs, optical discs, flash memories, or forms of EPROM or EEPROM. The instructions may be executed to support one or more aspects of the functionality described in this disclosure.

In addition, in some aspects, the functionality described herein may be provided within dedicated hardware and/or software modules. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components. Also, the techniques could be fully implemented in one or more circuits or logic elements. The techniques of this disclosure may be implemented in a wide variety of devices or apparatuses, including an IMD, an external programmer, a combination of an IMD and external programmer, an integrated circuit (IC) or a set of ICs, and/or discrete electrical circuitry, residing in an IMD and/or external programmer.

## Claims

1. A medical device system (2) configured to detect one or more coughs of a patient (4), the medical device system (2) comprising:
a medical device comprising:
a plurality of electrodes (16A, 16B) configured to collect an electrogram (EGM) signal, wherein the EGM signal includes noise indicative of one or more muscle movements that occur during a cough;
and
an accelerometer configured to collect an accelerometer signal,
wherein the accelerometer signal is indicative of one or more patient movements that occur during a cough; and
processing circuitry (14) configured to:
identify, in the EGM signal, a segment of the EGM signal including the noise indicative of one or more muscle movements occurring during a cough;
wherein the EGM signal comprises a first sequence of EGM samples, and wherein to identify the segment of the EGM signal, the processing circuitry is configured to:
generate a second sequence of EGM samples, wherein the second sequence of EGM samples represents a derivative of the first sequence of EGM samples;
identify, based on the second sequence of EGM samples, a set of slope reversals in a portion of the first sequence of EGM samples; and
determine if a number of slope reversals in the set of slope reversals is greater than a threshold number of slope reversals;
identify, in response to identifying the segment of the EGM signal, a segment of the accelerometer signal which is collected over a period of time, wherein the period of time in which the accelerometer signal is collected corresponds to a period of time in which the segment of the EGM signal is collected;
determine whether a parameter value associated with the segment of the accelerometer signal is greater than a threshold parameter value; and
increment, in response to the parameter value associated with the segment of the accelerometer signal being greater than the threshold parameter value, a cough count value.

2. The medical device system (2) of claim 1, wherein to identify the set of slope reversals, the processing circuitry (14) is further configured to:
select a portion of the second sequence of EGM samples corresponding to the portion of the first sequence of EGM samples;
identify a set of zero crossing events in the second sequence of EGM samples, wherein each zero crossing event of the set of zero crossing events represents an event in which a sign of a magnitude of a first EGM sample of the second sequence of EGM samples is different than a sign of a magnitude of a second EGM sample of the second sequence of EGM samples, wherein the second EGM sample is consecutive to the first EGM sample;
calculate an intensity value corresponding to each identified zero crossing event of the set of zero crossing events, wherein the intensity value represents a difference between the first EGM sample corresponding to the respective zero crossing event and the second EGM sample corresponding to the respective zero crossing event;
determine whether the intensity value corresponding to each identified zero crossing event of the set of zero crossing events is greater than a threshold intensity value; and
identify the set of slope reversals as including each zero crossing event of the set of zero crossing events where the respective intensity value is greater than the threshold intensity value.

3. The medical device system (2) of claim 1, wherein to identify the segment of the EGM signal, the processing circuitry (14) is further configured to:
calculate a median intensity value of the set of slope reversals;
determine whether the median intensity value of the set of slope reversals is greater than a threshold median intensity value; and
identify, if the number of slope reversals in the set of slope reversals is greater than the threshold number of slope reversals and if the median intensity value is greater than the threshold median intensity value, the segment of the second sequence of EGM samples as the segment of the EGM signal.

4. The medical device system (2) of claim 1, wherein to identify the segment of the EGM signal, the processing circuitry (14) is further configured to:
calculate a sum of the respective intensity values corresponding to each slope reversal of the set of slope reversals;
determine whether the sum is greater than a threshold sum value; and
identify, if the number of slope reversals in the set of slope reversals is greater than the threshold number of slope reversals and if the sum is greater than the sum value, the segment of the sequence of EGM samples as the segment of the EGM signal.

5. The medical device system (2) of claim 1, wherein to determine the set of slope reversals in the portion of the second sequence of EGM samples, the processing circuitry (14) is configured to:
detect, in the first sequence of EGM samples, an R-wave;
identify a window of EGM samples of the second sequences of EGM samples following the R-wave;
identify a set of slope change events within the window of EGM samples;
determine an intensity value corresponding to each slope change event of the set of slope change events; and
determine, in response to the intensity value corresponding to a respective slope change event of the set of slope change events being greater than a threshold intensity value, that the respective slope change event represents a slope reversal of the set of slope reversals.

6. The medical device system (2) of claim 1, wherein the processing circuitry (14) is further configured to:
determine, based on the cough count value, a cough rate associated with the patient over a period of time, wherein the cough rate represents a number of coughs detected per unit time; and
identify, in response to the cough rate increasing by a predetermined amount over the period of the time, an occurrence of a patient condition.

7. The medical device system (2) of claim 6, wherein the processing circuitry (14) is further configured to output an alert indicting the occurrence of the patient condition identified by the processing circuitry (14).

8. The medical device system (2) of any of the above claims, wherein the accelerometer signal represents a three-axis accelerometer signal comprising a vertical axis accelerometer signal vector, a lateral axis accelerometer signal vector, and a frontal axis accelerometer signal vector, and wherein to determine whether the parameter value associated with the segment of the accelerometer signal is greater than the threshold parameter value, the processing circuitry (14) is configured to:
determine whether a frontal parameter value associated with the frontal axis accelerometer signal vector is greater than a threshold frontal parameter value.

9. The medical device system (2) of any of the above claims, wherein the period of time in which the segment of the accelerometer signal is collected is the same as the period of time in which the segment of the EGM signal is collected.

10. The medical device system (2) of any of the above claims, wherein the period of time in which the segment of the accelerometer signal is collected overlaps with the period of time in which the segment of the EGM signal is collected.

11. A non-transitory computer-readable medium comprising instructions for causing one or more processors to:
receive an electrogram (EGM) signal acquired by a plurality of electrodes, wherein the EGM signal is indicative of one or more muscle movements that occur during a cough;
receive an accelerometer signal acquired by an accelerometer, wherein the accelerometer signal is indicative of one or more patient movements that occur during a cough;
identify, in the EGM signal, a segment of the EGM signal including the noise indicative of one or more muscle movements occurring during a cough;
wherein the EGM signal comprises a first sequence of EGM samples, and wherein to identify the segment of the EGM signal,
generate a second sequence of EGM samples, wherein the second sequence of EGM samples represents a derivative of the first sequence of EGM samples;
identify, based on the second sequence of EGM samples, a set of slope reversals in a portion of the first sequence of EGM samples; and
determine if a number of slope reversals in the set of slope reversals is greater than a threshold number of slope reversals;
identify, in response to identifying the segment of the EGM signal, a segment of the accelerometer signal which is collected over a period of time, wherein the period of time in which the accelerometer signal is collected corresponds to a period of time in which the segment of the EGM signal is collected;
determine whether a parameter value associated with the segment of the accelerometer signal is greater than a threshold parameter value; and
increment, in response to the parameter value associated with the segment of the accelerometer signal being greater than the threshold parameter value, a cough count value.

## Patentansprüche

1. Medizinisches Vorrichtungssystem (2), das dazu ausgelegt ist, einen oder mehrere Hustenstöße eines Patienten (4) zu detektieren, wobei das medizinische Vorrichtungssystem (2) umfasst:
eine medizinische Vorrichtung, umfassend:
eine Vielzahl von Elektroden (16A, 16B), die dazu ausgelegt ist, ein Elektrogramm(EGM)-Signal aufzufangen, wobei das EGM-Signal Rauschen einschließt, das eine oder mehrere Muskelbewegungen angibt, die während eines Hustenstoßes auftreten; und
einen Beschleunigungsmesser, der dazu ausgelegt ist, ein Beschleunigungsmessersignal aufzufangen, wobei das Beschleunigungsmessersignal eine oder mehrere Patientenbewegungen angibt, die während eines Hustenstoßes auftreten; und
eine Verarbeitungsschaltungsanordnung (14), die ausgelegt ist zum:
Identifizieren eines Segments des EGM-Signals in dem EGM-Signal, welches das Rauschen einschließt, das eine oder mehrere Muskelbewegungen angibt, die während eines Hustenstoßes auftreten;
wobei das EGM-Signal eine erste Sequenz von EGM-Abtastungen umfasst, und wobei zum Identifizieren des Segments des EGM-Signals die Verarbeitungsschaltungsanordnung ausgelegt ist zum:
Generieren einer zweiten Sequenz von EGM-Abtastungen, wobei die zweite Sequenz von EGM-Abtastungen eine Ableitung der ersten Sequenz von EGM-Abtastungen repräsentiert;
Identifizieren eines Satzes von Neigungsumkehrungen in einem Teil der ersten Sequenz von EGM-Abtastungen basierend auf der zweiten Sequenz von EGM-Abtastungen; und
Bestimmen, ob eine Anzahl von Neigungsumkehrungen in dem Satz von Neigungsumkehrungen größer als eine Schwellenanzahl von Neigungsumkehrungen ist;
Identifizieren eines Segments des Beschleunigungsmessersignals, das über eine Zeitdauer aufgefangen wird, in Reaktion auf Identifizieren des Segments des EGM-Signals, wobei die Zeitdauer, in der das Beschleunigungsmessersignal aufgefangen wird, einer Zeitdauer entspricht, in der das Segment des EGM-Signals aufgefangen wird;
Bestimmen, ob ein Parameterwert, der mit dem Segment des Beschleunigungsmessersignals assoziiert ist, größer als ein Schwellenparameterwert ist; und
in Reaktion darauf, dass der Parameterwert, der mit dem Segment des Beschleunigungsmessersignals assoziiert ist, größer als der Schwellenparameterwert ist, Inkrementieren eines Hustenstoßzählwerts.

2. Medizinisches Vorrichtungssystem (2) nach Anspruch 1, wobei die Verarbeitungsschaltungsanordnung (14) zum Identifizieren des Satzes von Neigungsumkehrungen ferner ausgelegt ist zum:
Auswählen eines Teils der zweiten Sequenz von EGM-Abtastungen, der dem Teil der ersten Sequenz von EGM-Abtastungen entspricht;
Identifizieren eines Satzes von Nulldurchgangsereignissen in der zweiten Sequenz von EGM-Abtastungen, wobei jedes Nulldurchgangsereignis des Satzes von Nulldurchgangsereignissen ein Ereignis repräsentiert, bei dem ein Vorzeichen einer Größe einer ersten EGM-Abtastung der zweiten Sequenz von EGM-Abtastungen sich von einem Vorzeichen einer Größe einer zweiten EGM-Abtastung der zweiten Sequenz von EGM-Abtastungen unterscheidet, wobei die zweite EGM-Abtastung der ersten EGM-Abtastung folgt;
Berechnen eines Intensitätswerts, der jedem identifizierten Nulldurchgangsereignis des Satzes von Nulldurchgangsereignissen entspricht, wobei der Intensitätswert eine Differenz zwischen der ersten EGM-Abtastung, die dem jeweiligen Nulldurchgangsereignis entspricht, und der zweiten EGM-Abtastung, die dem jeweiligen Nulldurchgangsereignis entspricht, repräsentiert;
Bestimmen, ob der Intensitätswert, der jedem identifizierten Nulldurchgangsereignis des Satzes von Nulldurchgangsereignissen entspricht, größer als ein Schwellenintensitätswert ist; und
Identifizieren des Satzes von Neigungsumkehrungen als jedes Nulldurchgangsereignis des Satzes von Nulldurchgangsereignissen einschließend, wo der jeweilige Intensitätswert größer als der Schwellenintensitätswert ist.

3. Medizinisches Vorrichtungssystem (2) nach Anspruch 1, wobei die Verarbeitungsschaltungsanordnung (14) zum Identifizieren des Segments des EGM-Signals ferner ausgelegt ist zum:
Berechnen eines Medianwerts des Intensitätswerts des Satzes von Neigungsumkehrungen;
Bestimmen, ob der Medianwert des Intensitätswert des Satzes von Neigungsumkehrungen größer als ein Schwellenmedianwert des Intensitätswerts ist; und
falls die Anzahl von Neigungsumkehrungen in dem Satz von Neigungsumkehrungen größer als die Schwellenanzahl von Neigungsumkehrungen ist, und falls der Medianwert des Intensitätswert größer als der Schwellenmedianwert des Intensitätswerts ist, Identifizieren des Segments der zweiten Sequenz von EGM-Abtastungen als das Segment des EGM-Signals.

4. Medizinisches Vorrichtungssystem (2) nach Anspruch 1, wobei die Verarbeitungsschaltungsanordnung (14) zum Identifizieren des Segments des EGM-Signals ferner ausgelegt ist zum:
Berechnen einer Summe der jeweiligen Intensitätswerte, die jeder Neigungsumkehr des Satzes von Neigungsumkehrungen entsprechen;
Bestimmen, ob die Summe größer als ein Schwellensummenwert ist; und
falls die Anzahl von Neigungsumkehrungen in dem Satz von Neigungsumkehrungen größer als die Schwellenanzahl von Neigungsumkehrungen ist, und falls die Summe größer als der Summenwert ist, Identifizieren des Segments der Sequenz von EGM-Abtastungen als das Segment des EGM-Signals.

5. Medizinisches Vorrichtungssystem (2) nach Anspruch 1, wobei die Verarbeitungsschaltungsanordnung (14) zum Bestimmen des Satzes von Neigungsumkehrungen in dem Teil der zweiten Sequenz von EGM-Abtastungen ausgelegt ist zum:
Detektieren einer R-Welle in der ersten Sequenz von EGM-Abtastungen;
Identifizieren eines Fensters von EGM-Abtastungen der zweiten Sequenzen von EGM-Abtastungen nach der R-Welle;
Identifizieren eines Satzes von Neigungsänderungsereignissen innerhalb des Fensters von EGM-Abtastungen;
Bestimmen eines Intensitätswerts, der jedem Neigungsänderungsereignis des Satzes von Neigungsänderungsereignissen entspricht; und
in Reaktion darauf, dass der Intensitätswert, der einem jeweiligen Neigungsänderungsereignis des Satzes von Neigungsänderungsereignissen entspricht, größer als ein Schwellenintensitätswert ist, Bestimmen, dass das jeweilige Neigungsänderungsereignis eine Neigungsumkehr des Satzes von Neigungsumkehrungen repräsentiert.

6. Medizinisches Vorrichtungssystem (2) nach Anspruch 1, wobei die Verarbeitungsschaltungsanordnung (14) ferner ausgelegt ist zum:
Bestimmen einer Hustenstoßrate, die mit dem Patienten assoziiert ist, über eine Zeitdauer basierend auf dem Hustenzählwert, wobei die Hustenstoßrate eine Anzahl von Hustenstößen repräsentiert, die pro Zeiteinheit detektiert werden; und
in Reaktion darauf, dass die Hustenstoßrate über die Zeitdauer um einen vorbestimmten Betrag zunimmt, Identifizieren eines Auftretens eines Patientenzustands.

7. Medizinisches Vorrichtungssystem (2) nach Anspruch 6, wobei die Verarbeitungsschaltungsanordnung (14) ferner dazu ausgelegt ist, einen Alarm auszugeben, der das Auftreten des durch die Verarbeitungsschaltungsanordnung (14) identifizierten Patientenzustands anzeigt.

8. Medizinisches Vorrichtungssystem (2) nach einem der obigen Ansprüche, wobei das Beschleunigungsmessersignal ein Dreiachsenbeschleunigungsmessersignal repräsentiert, das einen Vertikalachsen-Beschleunigungsmessersignalvektor, einen Querachsen-Beschleunigungsmessersignalvektor und einen Frontachsen-Beschleunigungsmessersignalvektor umfasst, und wobei die Verarbeitungsschaltungsanordnung (14) zum Bestimmen, ob der Parameterwert, der mit dem Segment des Beschleunigungsmessersignals assoziiert ist, größer als der Schwellenparameterwert ist, ausgelegt ist zum:
Bestimmen, ob ein Frontparameterwert, der mit dem Frontachsen-Beschleunigungsmessersignalvektor assoziiert ist, größer als ein Schwellenfrontparameterwert ist.

9. Medizinisches Vorrichtungssystem (2) nach einem der obigen Ansprüche, wobei die Zeitdauer, in der das Segment des Beschleunigungsmessersignals aufgefangen wird, die gleiche ist wie die Zeitdauer, in der das Segment des EGM-Signals aufgefangen wird.

10. Medizinisches Vorrichtungssystem (2) nach einem der obigen Ansprüche, wobei die Zeitdauer, in der das Segment des Beschleunigungsmessersignals aufgefangen wird, mit der Zeitdauer überlappt, in der das Segment des EGM-Signals aufgefangen wird.

11. Nichtflüchtiges computerlesbares Medium, das Anweisungen umfasst, um einen oder mehrere Prozessoren zu Folgendem zu veranlassen:
Empfangen eines Elektrogramm (EGM) -Signals, das durch eine Vielzahl von Elektroden erfasst wird, wobei das EGM-Signal eine oder mehrere Muskelbewegungen angibt, die während eines Hustenstoßes auftreten;
Empfangen eines Beschleunigungsmessersignals, das durch einen Beschleunigungsmesser erfasst wird, wobei das Beschleunigungsmessersignal eine oder mehrere Patientenbewegungen angibt, die während eines Hustenstoßes auftreten;
Identifizieren eines Segments des EGM-Signals in dem EGM-Signal, welches das Rauschen einschließt, das eine oder mehrere Muskelbewegungen angibt, die während eines Hustenstoßes auftreten;
wobei das EGM-Signal eine erste Sequenz von EGM-Abtastungen umfasst, und wobei zum Identifizieren des Segments des EGM-Signals ferner Folgendes geschieht:
Generieren einer zweiten Sequenz von EGM-Abtastungen, wobei die zweite Sequenz von EGM-Abtastungen eine Ableitung der ersten Sequenz von EGM-Abtastungen repräsentiert;
Identifizieren eines Satzes von Neigungsumkehrungen in einem Teil der ersten Sequenz von EGM-Abtastungen basierend auf der zweiten Sequenz von EGM-Abtastungen; und
Bestimmen, ob eine Anzahl von Neigungsumkehrungen in dem Satz von Neigungsumkehrungen größer als eine Schwellenanzahl von Neigungsumkehrungen ist;
Identifizieren eines Segments des Beschleunigungsmessersignals, das über eine Zeitdauer aufgefangen wird, in Reaktion auf Identifizieren des Segments des EGM-Signals, wobei die Zeitdauer, in der das Beschleunigungsmessersignal aufgefangen wird, einer Zeitdauer entspricht, in der das Segment des EGM-Signals aufgefangen wird;
Bestimmen, ob ein Parameterwert, der mit dem Segment des Beschleunigungsmessersignals assoziiert ist, größer als ein Schwellenparameterwert ist; und
in Reaktion darauf, dass der Parameterwert, der mit dem Segment des Beschleunigungsmessersignals assoziiert ist, größer als der Schwellenparameterwert ist, Inkrementieren eines Hustenstoßzählwerts.

## Revendications

1. Système de dispositif médical (2) configuré pour détecter une ou plusieurs toux d'un patient (4), le système de dispositif médical (2) comprenant :
un dispositif médical comprenant :
une pluralité d'électrodes (16A, 16B) configurées pour collecter un signal d'électrogramme (EGM), le signal EGM incluant un bruit indiquant un ou plusieurs mouvements musculaires qui se produisent pendant une toux ; et
un accéléromètre configuré pour collecter un signal d'accéléromètre, le signal d'accéléromètre indiquant un ou plusieurs mouvements de patient qui se produisent pendant une toux ; et
un circuit de traitement (14) configuré pour :
identifier, dans le signal EGM, un segment du signal EGM incluant le bruit indiquant un ou plusieurs mouvements musculaires qui se produisent pendant une toux ;
dans lequel le signal EGM comprend une première séquence d'échantillons d'EGM, et dans lequel pour identifier le segment du signal EGM, le circuit de traitement est configuré pour :
générer une seconde séquence d'échantillons d'EGM, la seconde séquence d'échantillons d'EGM représentant une dérivée de la première séquence d'échantillons d'EGM ;
identifier, sur la base de la seconde séquence d'échantillons d'EGM, un ensemble d'inversions de pente dans une partie de la première séquence d'échantillons d'EGM ; et
déterminer si un nombre d'inversions de pente dans l'ensemble d'inversions de pente est supérieur à un nombre seuil d'inversions de pente ;
identifier, en réponse à l'identification du segment du signal EGM, un segment du signal d'accéléromètre qui est collecté sur un laps de temps, le laps de temps pendant lequel est collecté le signal d'accéléromètre correspondant à un laps de temps pendant lequel est collecté le segment du signal EGM ;
déterminer si une valeur de paramètre associée au segment du signal d'accéléromètre est supérieure à une valeur de paramètre seuil ; et
incrémenter, en réponse au fait que la valeur de paramètre associée au segment du signal d'accéléromètre est supérieure à la valeur de paramètre seuil, une valeur de comptage de toux.

2. Système de dispositif médical (2) selon la revendication 1, dans lequel pour identifier l'ensemble d'inversions de pente, le circuit de traitement (14) est en outre configuré pour :
sélectionner une partie de la seconde séquence d'échantillons d'EGM correspondant à la partie de la première séquence d'échantillons d'EGM ;
identifier un ensemble d'événements de passage par zéro dans la seconde séquence d'échantillons d'EGM, chaque événement de passage par zéro de l'ensemble d'événements de passage par zéro représentant un événement dans lequel un signe d'une amplitude d'un premier échantillon d'EGM de la seconde séquence d'échantillons d'EGM est différent d'un signe d'une amplitude d'un second échantillon d'EGM de la seconde séquence d'échantillons d'EGM, dans lequel le second échantillon d'EGM est consécutif au premier échantillon d'EGM ;
calculer une valeur d'intensité correspondant à chaque événement de passage par zéro identifié de l'ensemble d'événements de passage par zéro, la valeur d'intensité représentant une différence entre le premier échantillon d'EGM correspondant à l'événement de passage par zéro respectif et le second échantillon d'EGM correspondant à l'événement de passage par zéro respectif ;
déterminer si la valeur d'intensité correspondant à chaque événement de passage par zéro identifié de l'ensemble d'événements de passage par zéro est supérieure à une valeur d'intensité seuil ; et
identifier l'ensemble d'inversions de pente comme incluant chaque événement de passage par zéro de l'ensemble d'événements de passage par zéro lorsque la valeur d'intensité respective est supérieure à la valeur d'intensité seuil.

3. Système de dispositif médical (2) selon la revendication 1, dans lequel pour identifier le segment du signal EGM, le circuit de traitement (14) est en outre configuré pour :
calculer une valeur d'intensité médiane de l'ensemble d'inversions de pente ;
déterminer si la valeur d'intensité médiane de l'ensemble d'inversions de pente est supérieure à une valeur d'intensité médiane seuil ; et
identifier, si le nombre d'inversions de pente dans l'ensemble d'inversions de pente est supérieur au nombre seuil d'inversions de pente et si la valeur d'intensité médiane est supérieure à la valeur d'intensité médiane seuil, le segment de la seconde séquence d'échantillons d'EGM comme étant le segment du signal EGM.

4. Système de dispositif médical (2) selon la revendication 1, dans lequel pour identifier le segment du signal EGM, le circuit de traitement (14) est en outre configuré pour :
calculer la somme des valeurs d'intensité respectives correspondant à chaque inversion de pente de l'ensemble d'inversions de pente ;
déterminer si la somme est supérieure à une valeur de somme seuil ; et
identifier, si le nombre d'inversions de pente dans l'ensemble d'inversions de pente est supérieur au nombre seuil d'inversions de pente et si la somme est supérieure à la valeur de somme, le segment de la séquence d'échantillons d'EGM comme étant le segment du signal EGM.

5. Système de dispositif médical (2) selon la revendication 1, dans lequel, pour déterminer l'ensemble d'inversions de pente dans la partie de la seconde séquence d'échantillons d'EGM, le circuit de traitement (14) est configuré pour :
détecter, dans la première séquence d'échantillons d'EGM, une onde R ;
identifier une fenêtre d'échantillons d'EGM des secondes séquences d'échantillons d'EGM suivant l'onde R ;
identifier un ensemble d'événements de changement de pente dans la fenêtre d'échantillons d'EGM ;
déterminer une valeur d'intensité correspondant à chaque événement de changement de pente de l'ensemble d'événements de changement de pente ; et
déterminer, en réponse au fait que la valeur d'intensité correspondant à un événement de changement de pente respectif de l'ensemble d'événements de changement de pente est supérieure à une valeur d'intensité seuil, que l'événement de changement de pente respectif représente une inversion de pente de l'ensemble d'inversions de pente.

6. Système de dispositif médical (2) selon la revendication 1, le circuit de traitement (14) est en outre configuré pour :
déterminer, sur la base de la valeur de comptage de toux, une fréquence de toux associée au patient sur un laps de temps, la fréquence de toux représentant un nombre de toux détectées par unité de temps ; et
identifier, en réponse à l'augmentation de la fréquence de toux d'une quantité prédéterminée sur le laps de temps, une occurrence d'une affection du patient.

7. Système de dispositif médical (2) selon la revendication 6, dans lequel le circuit de traitement (14) est en outre configuré pour émettre une alerte indiquant l'occurrence de l'état du patient identifié par le circuit de traitement (14).

8. Système de dispositif médical (2) selon l'une quelconque des revendications précédentes, dans lequel le signal d'accéléromètre représente un signal d'accéléromètre à trois axes comprenant un vecteur de signal d'accéléromètre d'axe vertical, un vecteur de signal d'accéléromètre d'axe latéral et un vecteur de signal d'accéléromètre d'axe frontal, et dans lequel pour déterminer si la valeur de paramètre associée au segment du signal d'accéléromètre est supérieure à la valeur de paramètre seuil, le circuit de traitement (14) est configuré pour :
déterminer si une valeur de paramètre frontal associée au vecteur de signal d'accéléromètre d'axe frontal est supérieure à une valeur de paramètre frontal seuil.

9. Système de dispositif médical (2) selon l'une quelconque des revendications précédentes, dans lequel le laps de temps pendant lequel le segment du signal d'accéléromètre est collecté est le même que le laps de temps pendant lequel le segment du signal EGM est collecté.

10. Système de dispositif médical (2) selon l'une quelconque des revendications précédentes, dans lequel le laps de temps pendant lequel le segment du signal d'accéléromètre est collecté chevauche le laps de temps pendant lequel le segment du signal EGM est collecté.

11. Support non transitoire lisible par ordinateur comprenant des instructions pour amener un ou plusieurs processeurs à :
recevoir un signal d'électrogramme (EGM) acquis par une pluralité d'électrodes, le signal EGM indiquant un ou plusieurs mouvements musculaires qui se produisent pendant une toux ;
recevoir un signal d'accéléromètre acquis par un accéléromètre, le signal d'accéléromètre indiquant un ou plusieurs mouvements du patient qui se produisent pendant une toux ;
identifier, dans le signal EGM, un segment du signal EGM incluant le bruit indiquant un ou plusieurs mouvements musculaires qui se produisent pendant une toux ;
dans lequel le signal EGM comprend une première séquence d'échantillons d'EGM, et dans lequel pour identifier le segment du signal EGM,
générer une seconde séquence d'échantillons d'EGM, la seconde séquence d'échantillons d'EGM représentant une dérivée de la première séquence d'échantillons d'EGM ;
identifier, sur la base de la seconde séquence d'échantillons d'EGM, un ensemble d'inversions de pente dans une partie de la première séquence d'échantillons d'EGM ; et
déterminer si un nombre d'inversions de pente dans l'ensemble d'inversions de pente est supérieur à un nombre seuil d'inversions de pente ;
identifier, en réponse à l'identification du segment du signal EGM, un segment du signal d'accéléromètre qui est collecté sur un laps de temps, le laps de temps pendant lequel est collecté le signal d'accéléromètre correspondant à un laps de temps pendant lequel est collecté le segment du signal EGM ;
déterminer si une valeur de paramètre associée au segment du signal d'accéléromètre est supérieure à une valeur de paramètre seuil ; et
incrémenter, en réponse au fait que la valeur de paramètre associée au segment du signal d'accéléromètre est supérieure à la valeur de paramètre seuil, une valeur de comptage de toux.
